(19) Europäisches Patentamt
European Patent Office
Office européen des brevets

(11) Publication number: 0 372 470 A2

# (12) EUROPEAN PATENT APPLICATION

(21) Application number: **89122376.0**

(22) Date of filing: **05.12.89**

(51) Int. Cl.[5]: **C07D 333/24, C07D 409/12, C07D 417/12, C07D 307/54, C07D 213/57, C07D 231/12, C07D 261/08, C07D 233/54, C07D 277/30, C07C 255/41, A61K 31/38**

(30) Priority: **08.12.88 US 281568**

(43) Date of publication of application: **13.06.90 Bulletin 90/24**

(84) Designated Contracting States: **AT BE CH DE ES FR GB GR IT LI LU NL SE**

(71) Applicant: **CIBA-GEIGY AG**
**Klybeckstrasse 141**
**CH-4002 Basel(CH)**

(72) Inventor: **Walker, Gordon N., Dr.**
**Lake Trail West Mount Kemble Lake**
**Morristown, NJ 07960(US)**

(74) Representative: **Zumstein, Fritz, Dr. et al**
**Bräuhausstrasse 4**
**D-8000 München 2(DE)**

## (54) Novel alpha-cyano-beta-oxopropionamides.

(57) α-(Substituted carbamoyl)-β-aryl- and heteroaryl-β-oxopropionitriles of formula

CN
|
A-CO- CH -CONH-B (I),

enol ethers thereof and salts thereof, as well as pharmaceutical preparations containing same and methods of preparing and using these compounds are disclosed. Said compounds represent novel antiinflammatory and antirheumatic agents. Their property to interfere with both the cyclooxygenase and lipoxygenase pathway of the arachidonic fatty acid bioconversion to inflammatory mediators make them valuable therapeutics. These properties render the mentioned substituted carbamoyl-β-oxopropionitriles useful for the treatment of arthritic and rheumatic diseases and other inflammatory conditions in mammals.

EP 0 372 470 A2

# NOVEL ALPHA-CYANO-BETA-OXOPROPIONAMIDES

Anilides and heterocyclylamides of α-cyano-β-oxo-β-pyrrolylpropionic acids are known to be antiinflammatory agents. U.S. Patent No. 4,256,759 discloses antiinflammatory, antiarthritic and immunomodulatory β-oxo-α-phenylcarbamoyl-β-pyrrolylpropionitriles, wherein the pyrrole nitrogen atom is substituted by lower alkyl or phenyl-lower alkyl. Related compounds wherein the phenylcarbamoyl group is replaced by a pyridylcarbamoyl or other heterocyclylcarbamoyl and heterocyclyl-lower alkyl-carbamoyl groups are described in U.S. Patent No. 4,435,407.

U.S. Patent No. 4,644,010 further discloses analgesic β-oxo-α-phenylcarbamoyl-β-pyrrolylpropionitriles, wherein the pyrrole nitrogen atom is unsubstituted. The related benzoylcyanoacetanilides and the corresponding o- and p-toluidides and p-anisidides have been described already by Dains and Griffin, J. Am. Chem. Soc. 35, 959 (1913), but without mentioning any physiological property.

There is still a need for other antiinflammatory agents which complement the properties of the valuable compounds described in the mentioned U.S. Patents. Such novel antiinflammatory and antirheumatic agents have now been found.

The present invention is concerned with α-(substituted carbamoyl)-β-aryl- and heteroaryl-β-oxopropionitriles of formula

$$A\text{-}CO\text{-}\overset{\overset{\displaystyle CN}{|}}{CH}\text{-}CONH\text{-}B \quad (I),$$

enol ethers thereof and salts thereof, as well as with pharmaceutical preparations containing same and methods of preparing and using these compounds. Said compounds represent novel antiinflammatory and antirheumatic agents. Their property to interfere with both the cyclooxygenase and lipoxygenase pathway of the arachidonic fatty acid bioconversion to inflammatory mediators make them valuable therapeutics. These properties render the mentioned substituted carbamoyl-β-oxopropionitriles useful for the treatment of arthritic and rheumatic diseases and other inflammatory conditions in mammals.

The present invention relates to the novel compounds of formula I, processes for preparing same, pharmaceutical compositions comprising said compounds, and methods of treating arthritic and rheumatic diseases and other inflammatory conditions by administering said compounds and compositions to mammals in need of same.

Particularly the invention relates to compounds of formula

$$A\text{-}CO\text{-}\overset{\overset{\displaystyle CN}{|}}{CH}\text{-}CONH\text{-}B \quad (I),$$

wherein A is selected from the group consisting of five- and six-membered unsubstituted or substituted aromatic heterocyclyl with the exception of pyrrolyl, five- and six-membered aromatic heterocyclyl-vinyl, and unsubstituted and substituted phenyl, and wherein B is unsubstituted or substituted phenyl, pyridyl or thiazolyl, with the proviso that when A is unsubstituted phenyl B does not represent unsubstituted phenyl, methylphenyl or methoxyphenyl, further to enol tautomers, lower alkyl enol ethers and salts thereof.

The general definitions used herein have the following meaning within the scope of the present invention.

The term "lower" referred to above and hereinafter in connection with organic radicals or compounds, respectively, defines such with up to and including 7, preferably up to and including 4, and advantageously one or two carbon atoms.

A lower alkyl group preferably contains 1-4 carbon atoms and represents, for example, ethyl, propyl, butyl or advantageously methyl.

A lower alkoxy group preferably contains 1-4 carbon atoms and represents, for example, ethoxy, propoxy, isopropoxy or advantageously methoxy.

Halogen preferably represents chloro or fluoro but may also be bromo or iodo.

A five- or six-membered aromatic heterocyclyl residue of the invention comprises, for example, one oxygen atom, one sulfur atom, one, two, three or four nitrogen atoms, or both one nitrogen atom and one oxygen or sulfur atom. Such a heterocyclyl residue is, for example, 2- or 3-furyl, 2- or 3-thienyl, 2- or 4-imidazolyl, 3-, 4- or 5-pyrazolyl, 1,2,4-triazolyl, tetrazolyl, 4- or 5-oxazolyl, 3-, 4- or 5-isoxazolyl, 2-, 4- or 5-thiazolyl, 2-, 3- or 4-pyridyl, pyrazinyl, 2-, 4- or 5-pyrimidinyl, or 3- or 4-pyridazinyl. The mentioned heterocycles may be substituted, for example at carbon atoms by one or more residues selected from lower alkyl, for example methyl, phenyl-lower alkyl, for example benzyl, phenyl, heterocyclyl, for example 2- or 3-furyl or 2- or 3-thienyl, and halogen, for example chloro or bromo, and at a nitrogen atom by lower alkyl, for example methyl, or phenyl-lower alkyl, for example benzyl. Examples for such substituted heterocyclyl residues are 5-methyl-2-furyl, 4- or 5-phenyl-2-furyl, 5-chloro-2-furyl, 5-bromo-2-furyl, 5-methyl-2-thienyl, 3,4-dimethyl-2-thienyl, 4- or 5-phenyl-2-thienyl, 5-(2-thienyl)-2-thienyl, 1-methyl-2- or 5-imidazolyl, 1-benzyl-2- or 5-imidazolyl, 1,3-dimethyl-5-pyrazolyl, 1,5-dimethyl-3-pyrazolyl, 5-methyl-3-isoxazolyl, 2-methyl- or phenyl-4-thiazolyl, 4-methyl- or phenyl-2-thiazolyl, 2,4-dimethyl-5-thiazolyl; 4-, 5- or 6-methyl-2-pyridyl, or 2,6-dimethyl-4-pyridyl.

A five- or six-membered aromatic heterocyclyl-vinyl group is preferably 2-substituted vinyl bearing one of the mentioned heterocyclyl residues, for example 2-(2-furyl)-vinyl, 2-(2-thienyl)vinyl, 2-(2-pyridyl)vinyl, and also 2-(2-pyrrolyl)vinyl or 2-(1-methyl-2-pyrrolyl)vinyl.

Substituted phenyl is a phenyl group bearing one, two, three, four or five, particularly one or two, substituents, for example selected from the group consisting of lower alkyl, for example methyl, phenyl, hydroxy, lower alkoxy, for example methoxy, halogen, for example chloro or fluoro, or trifluoromethyl. Such substituted phenyl is, for example, o-, m- or p-tolyl, 3,4-xylyl, 2,6-xylyl, p-biphenylyl, p-hydroxyphenyl, o- or p-methoxyphenyl, 3,4-dimethoxyphenyl, o-, m- or p-chlorophenyl, 2,4- or 3,4-dichlorophenyl, 3-chloro-4-methylphenyl, m- or p-fluorophenyl, 2,4-difluorophenyl, 3-chloro-4-fluorophenyl, 4-chloro-3-fluorophenyl, m- or p-trifluoromethylphenyl, or 4-chloro-3-trifluoromethylphenyl.

Enol tautomers of the compounds of formula I are in equilibrium with said compounds of formula I and may be represented by following formulae

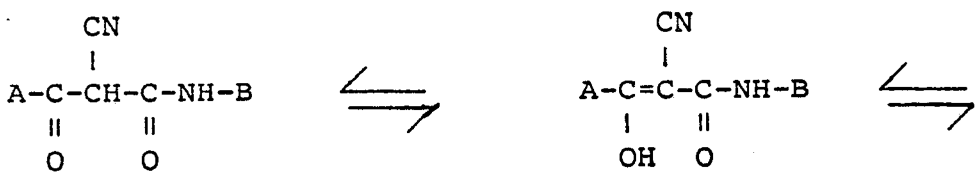

(I) (Ia)

wherein A and B have the meanings as previously defined for compounds of formula I. There are also other enol tautomers to be considered being in equilibrium with the basic structure shown in formula I, for example enol tautomers involving the carbamoyl carbonyl function, as in formula

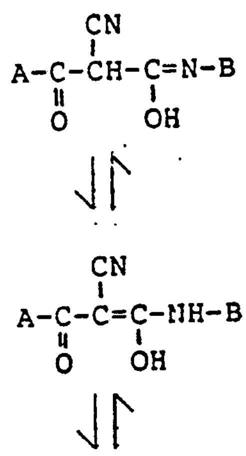

(Ib) and

(Ic),

and di-enolic tautomers of formula

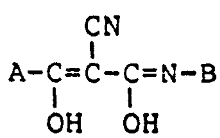

(Id).

Lower alkyl enol ethers of the invention are those wherein the hydrogen atom of an enolic hydroxy group in one of the mentioned enol tautomers is replaced by lower alkyl, for example ethyl or preferably methyl. In particular, an enol ether of the invention is a compound of above formula Ia, wherein the hydrogen atom of the hydroxy group is replaced by lower alkyl, for example methyl.

The compounds of formula I have acidic properties and readily form salts derived from enolic tautomers of formula Ia, Ic or Id with suitable inorganic or organic bases. Salts of compounds of formula I are especially pharmaceutically acceptable, non-toxic salts, for example alkali metal salts, for example sodium or potassium salts, alkaline earth metal salts, for example magnesium or calcium salts, zinc salts, ammonium salts, and in particular salts with organic amines, such as optionally hydroxy-substituted mono-, di- or trialkylamines, for example with 2-hydroxyethylamine, tris-(hydroxymethyl)methylamine, diethylamine, di-(2-hydroxyethyl)amine, triethylamine, N,N-dimethyl-N-(2-hydroxyethyl)amine, tri-(2-hydroxyethyl)amine or N-methyl-D-glucamine, with cyclic amines, for example pyrrolidine or morpholine, or with corresponding diamines, for example ethylenediamine. Salts of compounds of formula I include also various hydrated salts thereof.

Compounds of formula I wherein the radical A is a heterocyclyl residue basic in nature also form acid addition salts. Said acid addition salts preferably are pharmaceutically acceptable, non-toxic salts, for example salts with strong mineral acids, for example hydrohalic, e.g. hydrochloric or hydrobromic acid, sulfuric, phosphoric, nitric or perchloric acid; with aliphatic or aromatic carboxylic acids, e.g. formic, acetic, propionic, succinic, glycolic, lactic, malic, tartaric, gluconic, citric, ascorbic, maleic, fumaric, hydroxymaleic, pyruvic, phenylacetic, benzoic, 4-aminobenzoic, anthranilic, 4-hydroxybenzoic, salicylic, 4-aminosalicyclic, pamoic, or nicotinic acid; or with sulfonic acids, e.g. methanesulfonic, ethanesulfonic, hydroxyethanesulfonic, benzenesulfonic, p-toluenesulfonic, naphthalenesulfonic, sulfanilic or cyclohexylsulfamic acid.

Compounds of formula I wherein the radical A is a basic heterocyclyl residue may also form inner salts.

The compounds of the invention exhibit valuable pharmacological properties, primarily antiinflammatory, analgesic (antinociceptive), antirheumatic, and antiarthritic activity. These can be demonstrated by in vitro or in vivo tests, using for the latter advantageously mammals, such as rats, mice, guinea pigs or dogs, as test objects. The compounds of the invention can be adminstered to the animals either enterally, preferably orally, parenterally, e.g. subcutaneously or intravenously, or topically, for example, in the form of aqueous or oily solutions or starchy suspensions. The applied dosage may range between about 0.1 and 100 mg/kg/day, preferably between about 1 and 50 mg/kg/day, advantageously between about 5 and 25 mg/kg/day. The tests chosen are among the classical assay methods for said activities, such as the carrageenin paw-edema, or adjuvant arthritis tests in rats, the canine synovitis or ultraviolet erythema assays, or more recent tests, such as neutral protease inhibition, inhibition of leukocyte chemotaxis, decrease of neutrophil adherence, inhibition of prostaglandin synthetase or cyclooxygenase, inhibition of 5-lipoxygenase, the phenylquinone writhing test in mice, or inhibition of cartilage matrix degradation.

The carrageenin paw-edema assay for antiinflammatory activity is carried out in rats as follows:

One hour after compounds are administered orally, 0.1 ml of carrageenin (1%) is injected into plantar area of one hind paw. Difference of swelling is measured between contralateral and injected paw by means of mercury displacement at designated times. Compounds of the invention at a dose of 100 mg/kg p.o. usually afford protection against carrageenin-induced edema in rats measured 3 hours after administration in the range of 20% up to 60%.

The established adjuvant arthritis test for antiarthritic activity is performed essentially as described in Proc. Soc. Biol. Med. 137, 506 (1971). Several compounds of the invention are effective in the established adjuvant arthritis test in the rat affording 30% to 70% protection when administered at a dose ranging from 10 to 25 mg/kg p.o.

The compounds of the invention demonstrate dual 5-lipoxygenase/cyclooxygenase inhibitory activity. Most non-steroidal antiinflammatory drugs (NSAIDs) including some of the previously disclosed $\beta$-oxo-$\alpha$-phenylcarbamoyl-$\beta$-pyrrolylpropionitriles are known to block selectively the cyclooxygenase pathway thereby prohibiting or at least slowing down the formation of prostaglandins, thromboxanes and prostacyclins from arachidonic acid. This inhibitory action on cyclooxygenase produces the desired antiinflammatory effect but at the same time may increase the formation of leukotrienes by the alternative arachidonic acid metabolism in the lipoxygenase pathway. Leukotrienes are a group of mediators with potent leukocyte-chemoattractant, smooth muscle-constricting and vascular permeability-enhancing properties. Leukotrienes have been implicated in the pathogenesis of a variety of vascular and pulmonary disorders involving leukocyte and smooth muscle activation.

Prostaglandin synthetase inhibition is determined essentially by assaying in vitro $PGE_2$ formation from arachidonate as described by Takeguchi et al., Biochemistry 10, 2372 (1971) and by Tomlinson et al., Biochem. Biophys. Res. Commun. 46, 552 (1972). Cyclooxygenase inhibition may also be measured by the radiometric assay described in Ku et al., Biochem. Pharmacol. 24, 641 (1974).

5-Lipoxygenase inhibition is determined e.g. by measuring the percent inhibition of the synthesis of 5-HETE [(5S)-5-hydroxy-6,8,11,14-eicosatetraenoic acid] and leukotriene $B_4$ ($LTB_4$, 5,12-dihydroxy-6,8,10,14-eicosatetraenoic acid) in ionophore A-23187-stimulated guinea pig polymorphonuclear leukocytes, essentially according to the procedure described by Borgeat and Samuelsson, Proc. Natl. Acad. Sci. USA 76, 2148 (1979), using $^{14}C$-arachidonic acid. $IC_{50}$ values are determined graphically as the concentration of test compound at which the synthesis of 5-HETE and $LTB_4$-like products is reduced to 50% of their respective control values.

Compounds of the invention are usually highly effective inhibitors of prostaglandin synthetase as determined in the bull seminal vesicle microsome enzyme assay, with 50% inhibition concentrations ($IC_{50}$) in the range of 1 to 100 $\mu$M. At the same time these compounds show $IC_{50}$ in the range of 1 to 100 $\mu$M for 5-lipoxygenase inhibition as measured in vitro in A-23187-stimulated guinea pig polymorphonuclear leukocytes.

Further to that the compounds of the invention reduce neutrophil activation, a property that is indicative of antiarthritic activity, e.g. in the treatment of rheumatoid arthritis.

Inhibition of leukocyte chemotaxis may be measured as described in Ann. N.Y. Acad. Sci. 256, 177 (1975). The inhibition of the chemotactic activation of neutrophils is also determined in vitro, e.g. by measuring the inhibition of the binding of f-MLP (formyl-methionyl-leucyl-phenyl-alanine) to human neutrophils in vitro as follows: Human neutrophils are isolated by a modification of the procedure of Ferrante and Thong, J. Immunol. Methods 24, 389 (1978). Red cells remaining in the neutrophil preparation are lysed by separate treatments with 0.83% $NH_4Cl$ and 0.1 M tris-HCl, pH 7.5. Neutrophils are incubated in Hanks' buffer at 0 °C for 60 minutes with 15 nM $^3H$-f-MLP and test compound. Aliquots of the incubates are filtered and total $^3H$-f-MLP bound is measured. Non-specific binding in the presence of excess non-radioactive f-MLP is subtracted, and percent inhibition of binding by the test drug is calculated. $IC_{50}$ of compounds of the invention inhibiting binding of f-MLP to human neutrophils are in the range of 1 to 20 $\mu$M.

The phenyl-p-benzoquinone-induced writhing test for analgesic activity, described in J. Pharmacol. Exp. Thera. 125, 237 (1959) is performed in mice as follows: Mice previously fasted overnight are treated orally with the test compound dissolved in 0.75% methylcellulose at doses of 1 to 50 mg/kg, or with the vehicle alone, 55 minutes before the induction of the writhing syndrome by i.p. injection of 0.25 ml of a suspension of phenyl-p-benzoquinone (0.03%) in tragacanth (0.4%). Starting 5 minutes, thereafter, the number of writhing movements provoked by the irritant are counted over an observation period of 10 minutes. The analgesic (antinociceptive) $ED_{50}$ of the test compound is the dose which reduces the mean frequency of writhing movements by 50% in comparison to the controls.

The compounds of the invention are also active in the cartilage-synovium co-culture model of cartilage matrix degradation, indicative of their effectiveness in osteoarthritis. The screen is carried out as follows: The proteoglycan matrix of bovine nasal septum cartilage is labeled in vitro by incorporation of $^{35}S$ into glycosaminoglycan. Cartilage slices are incubated overnight in a sulfate-free medium containing $^{35}S$-sodium sulfate. $^{35}S$-Labeled cartilage slices are co-cultured with normal synovium explants in multiwell tissue culture plates. After 4 days incubation a 100 $\mu$l aliquot of medium is counted. Cartilage slices are hydrolyzed and a 100 $\mu$l aliquot of cartilage hydrolysate is counted. The percent $^{35}S$ released into the medium is determined and the percent of inhibition of matrix degradation is calculated.

The aforementioned advantageous properties render the compounds of the invention useful as antiinflammatory, analgesic and antiarthritic agents especially for the treatment and amelioration of e.g. pain and inflammatory disorders, such as rheumatoid arthritis and osteoarthritis in mammals, including man.

Particularly useful are compounds of formula I, enol tautomers, lower alkyl enol ethers and salts thereof, wherein

A is selected from the group consisting of five membered aromatic heterocyclyl comprising one oxygen atom, one sulfur atom, or two heteroatoms wherein one is nitrogen and the other one is nitrogen, oxygen or sulfur, and wherein the aromatic heterocycle is unsubstituted or substituted at carbon atoms by one, two or three residues selected from lower alkyl, phenyl, furyl, thienyl and halogen, and at a nitrogen atom by lower alkyl; six-membered aromatic heterocyclyl comprising one or two nitrogen atoms, unsubstituted or substituted at carbon atoms by lower alkyl or phenyl; furylvinyl; thienylvinyl; and phenyl substituted by one or two residues selected from lower alkyl, phenyl, hydroxy, lower alkoxy, halogen and trifluoromethyl; and

B is selected from the group consisting of phenyl; phenyl substituted by one or two residues selected from lower alkyl, phenyl, hydroxy, lower alkoxy, halogen and trifluoromethyl; pyridyl; and thiazolyl.

Equally useful are compounds of formula I, enol tautomers, lower alkyl enol ethers and salts thereof, wherein A is phenyl and B is phenyl substituted by one or two residues selected from halogen and trifluoromethyl.

Preferred are compounds of formula I, enol tautomers, lower alkyl enol ethers and salts thereof, wherein A is five- or six-membered aromatic heterocyclyl selected from the group consisting of furyl, thienyl, imidazolyl, pyrazolyl, isoxazolyl, thiazolyl, pyridyl, pyrazinyl and pyrimidinyl, unsubstituted or substituted by $C_1$-$C_4$-alkyl, di-$C_1$-$C_4$-alkyl or phenyl; 2-furylvinyl; 2-thienylvinyl; or phenyl substituted by one or two

residues selected from $C_1$-$C_4$-alkyl, $C_1$-$C_4$-alkoxy, fluoro, chloro and trifluoromethyl; and B is phenyl unsubstituted or substituted by one or two residues selected from $C_1$-$C_4$-alkyl, $C_1$-$C_4$-alkoxy, fluoro, chloro and trifluoromethyl, or is pyridyl or thiazolyl.

Particularly preferred are compounds of formula I, enol tautomers thereof and salts thereof, wherein A is 2- or 3-furyl, 5-bromo-2-furyl, 2- or 3-thienyl, 2-(2-furyl)vinyl, or 2-(2-thienyl)vinyl; and B is phenyl or phenyl substituted by one or two residues selected from $C_1$-$C_4$-alkyl, $C_1$-$C_4$-alkoxy, fluoro, chloro and trifluoromethyl, or is 2-pyridyl or 2-thiazolyl.

Likewise preferred are compounds of formula I, enol tautomers thereof and salts thereof, wherein A is 2- or 4-imidazolyl, 1-methyl-2- or 5-imidazolyl, 3-pyrazolyl, 1,5-dimethyl-3-pyrazolyl, 1,3-dimethyl-5-pyrazolyl, 3- or 5-isoxazolyl, 5-methyl-3-isoxazolyl, 3-methyl-5-isoxazolyl, 2-, 4- or 5-thiazolyl, 2-methyl- or phenyl-4-thiazolyl, 2,4-dimethyl-5-thiazolyl, or 2-, 3- or 4-pyridyl, and B is phenyl unsubstituted or substituted by one or two residues selected from $C_1$-$C_4$-alkyl, $C_1$-$C_4$-alkoxy, fluoro, chloro and trifluoromethyl.

Likewise preferred are compounds of formula I, enol tautomers thereof and salts thereof, wherein A is phenyl substituted by $C_1$-$C_4$-alkyl, $C_1$-$C_4$-alkoxy, fluoro or chloro, and B is phenyl unsubstituted or substituted by one or two residues selected from $C_1$-$C_4$-alkyl, $C_1$-$C_4$-alkoxy, fluoro, chloro and trifluoromethyl, or wherein A is phenyl and B is phenyl substituted by one or two residues selected from fluoro and chloro.

Most preferred are compounds of formula I, enol tautomers thereof and salts thereof, wherein A is 2-furyl or 2-thienyl and B is phenyl, chlorophenyl, fluorophenyl, trifluoromethylphenyl, dichlorophenyl, difluorophenyl, or chlorofluorophenyl.

Highly preferred are the compounds of formula I described in the Examples, enol tautomers thereof and salts thereof.

The present invention also relates to processes for the manufacture of compounds of formula I and salts thereof. These can be prepared according to methods known per se to those skilled in the art, for example by

(a) condensing a compound of formula

A-CO-$CH_2$CN (II),

or a salt thereof, with an isocyanate of formula

B-N=C=O (III),

wherein A and B have the meaning as previously defined; or

(b) condensing a compound of formula

CN

A-CO-CH-COOH (IV),

or a functional carboxyl derivative thereof, with an amine of formula

B-$NH_2$ (V),

wherein A and B have the meaning as previously defined; or

(c) condensing a carboxylic acid of formula

A-COOH (VI),

or a functional derivative thereof, with a compound of formula

NC-$CH_2$-CONH-B (VII)

or a salt thereof, wherein A and B have the meaning as previously defined; or

(d) hydrolyzing an isoxazole of the formula

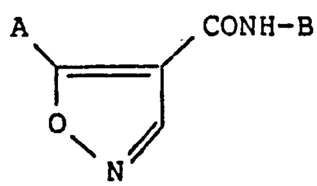

(VIII),

wherein A has the meaning as previously defined except isoxazolyl and B has the meaning as previously defined; or

(e) cleaving a conventional protecting group from a compound of the formula

CN

A′-CO-CH-CONH-B′ (IX),

wherein A′ has the meaning of A, represents five-membered nitrogen-containing heterocyclyl wherein the hydrogen atom bound to the nitrogen atom is replaced by a conventional amino protecting group, or represents hydroxy-substituted phenyl wherein the hydroxy group is protected by a conventional protecting

group; and wherein B′ has the meaning of B or represents hydroxy-substituted phenyl wherein the hydroxy group is protected by a conventional protecting group; and wherein at least one of A′ and B′ are different from A and B, respectively; and

optionally converting a resulting compound of formula I into another compound of formula I according to the definition; and

optionally converting a resulting compound of formula I into a lower alkyl enol ether thereof, or converting a resulting compound of formula I into a salt thereof, or converting a resulting salt of a compound of formula I into the free compound or into another salt thereof.

The condensation according to process (a) of the substituted β-oxopropionitrile of formula II with an isocyanate of formula III may be carried out under a variety of reaction conditions, without a solvent or in the presence of an unpolar or a non-protic, inert polar solvent in a temperature range between 0° C to 200° C, optionally in the presence of an inorganic or organic base.

Useful solvents are, for example, hydrocarbon solvents, e.g. benzene, toluene, xylene, or cyclohexane, ethers, e.g. diethyl ether, tetrahydrofuran, dimethoxyethane, or dioxane, esters, e.g. ethyl acetate, amides, e.g. dimethyl formamide or N-methylpyrrolidone, nitriles, e.g. acetonitrile, or other dipolar aprotic solvents, e.g. dimethylsulfoxide. Preferably the reaction is run in the presence of an inorganic base, for example sodium or potassium hydride, sodium or potassium carbonate or the like, or in the presence of an organic base, particularly a tertiary amine, e.g. triethylamine, tributylamine or dimethylaniline, or a pyridine base, e.g. pyridine. The addition of an inorganic or organic base lowers the reaction temperature considerably, for example to a range of 0° C to about 50° C, preferably around room temperature. Otherwise temperatures between 80° C and 150° C are contemplated, for example at the boiling temperature of the solvent used.

Salts of substituted β-oxopropionitriles of formula II used in process (a) are particularly alkali metal salts, for example sodium or potassium salts, or earth alkali metal salts, for example magnesium salts, or else ammonium salts. If the mentioned salts are used in the process, the preferred temperature range is 0° C to about 50° C without added extra base. Polar solvents such as dimethyl formamide or dimethylsulfoxide are preferred when using salts.

Process (a) is preferably performed in the following way: The substituted β-oxopropionitrile of formula II is treated with a slight molar excess of an anhydrous tri-lower alkylamine, preferably triethylamine, in one of the above-mentioned solvents, and then a molar equivalent of the appropriate isocyanate of formula III is added, or a solution thereof in the mentioned solvents. After stirring for about 2-24 hours at room temperature, the reaction mixture is reduced in volume by evaporation without excessive warming. The residue is treated with an excess of dilute aqueous acid, e.g. 0.1-0.3 N hydrochloric acid, and the crude products are extracted or collected, washed with water, dried, triturated and/or recrystallized from appropriate solvents, such as lower alkanols, alkanones, dialkyl ethers and/or alkyl alkanoates, e.g. methanol, acetone, diethyl ether and/or ethyl acetate.

Starting materials of formula II or III are either known or are prepared according to methods well known in the art. For example, β-oxopropionitriles of formula II are obtained by condensation of acetonitrile with corresponding carboxylic esters in the presence of e.g. sodium ethoxide, sodium methoxide or sodium hydride, in polar solvents such as the corresponding alcohol, dimethoxyethane or dimethyl formamide, or mixtures thereof with toluene or other hydrocarbon solvents. Alternatively, β-oxopropionitriles of formula II may be formed by condensation of ethyl formate with the corresponding methyl ketone, cyclocondensation of the obtained β-substituted β-oxopropionaldehyde to an isoxazole, and cleavage of the isoxazole with sodium hydroxide solution. Further known methods for the preparation of β-oxopropionitriles of formula II include thermolytic decarboxylative cleavage of 5-substituted isoxazole-3-carboxylic acids, substitution of chlorine by cyanide in chloromethylketones, or mixed condensation of an (aryl- or heteroaryl-)nitrile with acetonitrile followed by acid hydrolysis of the obtained β-substituted β-aminoacrylonitrile.

Process (b) involving reaction of a carboxylic acid of formula IV or a functional derivative thereof with a primary amine of formula V is carried out in a manner well-known. Suitable functional derivatives are carboxylic acid chlorides, anhydrides, for example symmetric anhydrides or mixed anhydrides with formic acid, acetic acid or preferably carbonic acid semi-esters, or esters, for example lower alkyl esters, preferably methyl or ethyl esters. Further suitable functional derivatives are those disclosed in U.S. Pat. No. 4,727,060 for the formation of an amide bond, for example reactive esters or reactive cyclic amides.

If a carboxylic acid chloride or anhydride is used as the functional derivative of a compound of formula IV, the condensation is carried out with an excess of the amine of formula V or with an equivalent amount of said amine and an equivalent or excess amount of another, non-reactive base, for example a tertiary amine, e.g. a tri-lower alkylamine such as triethylamine, or pyridine, in order to neutralize the generated acid. The reaction is carried out without solvent in an excess of the amine or in an unpolar or an aprotic polar solvent such as those mentioned under process (a), for example in an ether, e.g. diethyl ether, tetrahydrofuran or

dioxane, or in acetonitrile or a like dipolar aprotic solvent. Protic solvents, for example alcohols, e.g. ethanol or isopropanol, may also be used, but are less desirable. The condensation is performed at temperatures between -30° C and +80° C depending on the reactivity of the acid derivative used, preferably between 0° C and room temperature.

If a carboxylic ester is used as the functional derivative of a compound of formula IV, the condensation is carried out at a higher temperature, for example between 80° C and 200° C. For example, a high-boiling hydrocarbon solvent is used such as benzene, toluene or xylene, and the reaction is conducted at the boiling point of said solvent in order to remove the alkanol generated in the condensation of the ester with the amine.

The condensation using a free carboxylic acid of formula IV is preferably carried out in the presence of a condensing agent known in peptide chemistry, for example as described in U.S. Pat. No. 4,727,060, e.g. dicyclohexylcarbodiimide or 1,1′-carbonyldiimidazole.

Starting materials of formula IV are known. If they are novel, they are prepared by methods known in the art, for example by acylation of a sodium or potassium salt of a cyanoacetic ester with a carboxylic acid chloride or anhydride, e.g. a mixed anhydride with a carbonic acid semi-ester, in the presence of an inert, unpolar or aprotic dipolar solvent. For example, the sodium salt of ethyl cyanoacetate obtainable from ethyl cyanoacetate and sodium hydride is acylated with a suitable acid chloride or mixed anhydride with carbonic acid mono-ethyl ester in dimethoxyethane or a like polar solvent. Alternatively, free acids of formula IV may be obtained by carboxylation of corresponding β-oxopropionitriles with magnesium dimethyl carbonate, prepared by carbonation of magnesium methoxide in dimethyl formamide. Ester derivatives of compounds of formula IV are also available by reaction of β-substituted β-oxopropionitriles with chloroformates in the presence of base.

The condensation reaction of process (c) is carried out under conditions generally known for acylations of β-ketonitriles or β-ketoesters. Useful functional derivatives of a carboxylic acid of formula VI are the corresponding acid chlorides and acid anhydrides, for example symmetrical anhydrides or mixed anhydrides with e.g. formic acid, acetic acid or carbonic acid lower alkyl semi-esters, activated esters, for example succinimido, phthalimido, 4-nitrophenyl or vinyl esters, or activated amides, for example imidazolides or 3,5-dimethylpyrazolides. Useful salts of compounds of formula VII are alkali metal salts, for example lithium, sodium, potassium or cesium salts, earth alkali metal salts, for example magnesium salts, or thallium salts. Preferably the condensation reaction is performed using the mentioned acid chloride or mixed anhydride derivatives of the acid of formula VI and an alkali metal salt of the β-ketonitrile of formula VII. The reaction is carried out in one of the solvents mentioned under process (a), preferably in a polar ether, for example in dimethoxyethane, tetrahydrofuran or dioxane, in an inert dipolar aprotic solvent, for example in dimethyl formamide, N-methylpyrrolidone or dimethylsulfo xide, or in mixtures of said solvents with each other or with a hydrocarbon solvent, for example with toluene, at temperatures between 0° C and 100° C, preferably between 20° C and 50° C, for example around room temperature. The acylation reaction of process (c) may also be carried out using the mentioned acid chloride and the β-ketonitrile of formula VII in the presence of an activating base, for example 4-dimethylaminopyridine, or using the free carboxylic acid in the presence of a condensing agent, for example diethyl phosphorocyanidate, and in the presence of a base, for example triethylamine, in the mentioned preferred solvents and at the temperatures given.

Starting materials of formula VII are either known or are prepared by methods known in the art. For example they are obtained by reaction of cyanoacetic acid or a functional derivative thereof with the corresponding amine of formula V under the conditions mentioned under process (b) for the formation of an amide.

The hydrolysis of an isoxazole in process (d) is carried out using strong inorganic or organic bases, for example aqueous alkali metal hydroxides, e.g. lithium, sodium or potassium hydroxide, or quaternary ammonium hydroxides, e.g. benzyltrimethylammonium hydroxide. The isoxazoles of formula VIII are cleaved in the mentioned aqueous strong bases at temperatures between room temperature and 100° C, preferably between 50° C and 80° C. An organic co-solvent may be added to the reaction mixture to enhance solubility and thereby lowering the required reaction temperature, for example a lower alkanol, e.g. methanol or ethanol, or a water-miscible ether, e.g. dimethoxyethane.

The isoxazole starting materials of formula VIII are formed by reactions known in the art, for example by cyclocondensation of the corresponding α-formyl- or α-aminomethylene-β-oxopropionamides with hydroxylamine hydrochloride in aqueous methanol. The required tricarbonyl compounds or their equivalents in turn are available by an acylation reaction similar to the one described in process (c), for example by formylation of the corresponding β-substituted β-oxopropionamide with a suitable formic acid ester, or by equivalent aminomethylenation using, for example, a dimethyl formamide acetal, N-phenylformamidine, or a mixture of a primary amine and a formic acid orthoester. The hydrolysis of an isoxazole in process (d) can also be

performed under the specified conditions with concomitant ester hydrolysis and decarboxylation starting with a corresponding isoxazole-3-carboxylic ester, for example a methyl or ethyl ester. The isoxazole-3-carboxylic ester starting materials are formed by cyclocondensation of the corresponding α-methoxalyl- or α-ethoxalyl-β-oxopropionamides with hydroxylamine hydrochloride. These methoxalyl or ethoxalyl derivatives are available by ester condensation of methyl or ethyl oxalate and corresponding β-substituted β-oxopropionamides.

Cleaving a conventional protecting group from a compound of formula IX in process (e) is carried out by methods well-known in the art, depending on the type of protecting group present. Conventional protecting groups and corresponding deprotection reactions are described, for example in standard works, such as in J.F.W. McOmie, "Protective Groups in Organic Chemistry", Plenum Press, London and New York 1973, in Th. W. Greene, "Protective Groups in Organic Synthesis", Wiley, New York 1981, in "The Peptides", volume 3 (edited by E. Gross and J. Meienhofer), Academic Press, London and New York 1981, and in "Methoden der organischen Chemie", Houben-Weyl, 4th edition, vol. 15/1, Georg Thieme Verlag, Stuttgart 1974.

For example, benzyl, diphenylmethyl, trityl, benzyloxycarbonyl and substituted derivatives of said protecting groups are cleaved by hydrogenolysis, i.e. by treatment with hydrogen in the presence of a suitable hydrogenation catalyst, such as a palladium catalyst. 2-Halo-lower alkoxycarbonyl, e.g. 2,2,2-trichloroethoxycarbonyl, or aroylmethoxycarbonyl protecting groups are removed by metallic reducing agents, for example zinc in the presence of acetic acid. Diphenylmethoxycarbonyl, tert.-lower alkoxycarbonyl, e.g. tert.-butoxycarbonyl, or the hydroxyl-protecting groups tert.-lower alkyl, e.g. tert.-butyl; substituted oxymethyl, e.g. methoxymethyl, ethoxymethyl or 1-ethoxyethyl; substituted thiomethyl, e.g. methylthiomethyl; or 2-oxacycloalkyl, e.g. 2-tetrahydropyranyl or 2-tetrahydrofuryl, are cleaved with an acid, for example a mineral acid, e.g. hydrochloric acid; an organic carboxylic acid, e.g. formic acid, acetic acid or trifluoroacetic acid; an organic sulfonic acid, e.g. p-toluenesulfonic acid; or an ion exchange resin in acid form; optionally in the presence of water or an organic solvent, for example a lower alkanol, e.g. methanol; an ether, e.g. diethyl ether or tetrahydrofuran; or a chlorinated hydrocarbon, e.g. methylene chloride or chloroform. A silyl protecting group, for example trimethylsilyl, tert.-butyldimethylsilyl or 2-trimethylsilylethoxycarbonyl, is cleaved by the mentioned acids, or preferably, by fluoride anions, for example by sodium fluoride, potassium fluoride or a quaternary ammonium fluoride, e.g. tetraethylammonium fluoride or tetrabutylammonium fluoride, in a polar solvent, for example dimethylsulphoxide, dimethyl formamide or acetonitrile, optionally in the presence of a crown ether. The cleavage of protecting groups is usually carried out at low or ambient temperature, for example between 0 °C and room temperature, e.g. around 20 °C.

If A′ and B′ both represent protected residues A and B, respectively, the protecting groups may be cleaved in one step or consecutively, depending on the kind of protecting group present.

The starting material of formula IX is prepared according to any of the mentioned processes (a), (b), (c) or (d), using the corresponding compounds II to VIII, respectively, wherein the residue A is replaced by A′ and/or B is replaced by B′. The corresponding protected compounds are available according to methods described in the above-mentioned standard works on protective groups in organic chemistry.

The compounds of the invention, so obtained, can be converted into each other according to methods known per se. In particular, in a compound of formula I wherein A is five-membered heterocyclyl with at least one nitrogen atom bearing hydrogen, this nitrogen atom can be alkylated, for example methylated or benzylated, using conventional alkylating agents, for example alkyl halides or sulfonates, e.g. methyl iodide, methyl p-toluenesulfonate or benzyl bromide. In compounds wherein A and/or B is phenyl substituted by hydroxy, this substituent can be converted to alkoxy by the mentioned alkylating agents. Furthermore, in compounds wherein A and/or B is phenyl substituted by lower alkoxy, e.g. methoxy, this substituent can be converted to hydroxy using suitable acids, for example hydroiodic acid or, preferably, boron tribromide.

The compounds of the invention, so obtained, can be converted into lower alkyl enol ethers thereof according to methods known in the art. In particular, methyl enol ethers are formed from compounds of formula I and diazomethane, for example in etheral solution at around 0 °C. Otherwise, the compounds of the invention are converted into alkali metal salts, for example sodium salts, as described further below, and the salts treated with lower alkyl halides or sulfonates, for example methyl iodide or ethyl p-toluenesulfonate, in a polar solvent at temperatures between 0° and 50 °C, for example around room temperature.

The compounds of the invention can be converted into salts thereof by conventional means well-known in the art, for example by treatment with aqueous alkali metal or earth alkali metal hydroxide, preferably in stoichiometric amounts or with a slight excess, advantageously in the presence of an organic co-solvent, for example a lower alkanol, e.g. ethanol or methanol, or an ether, e.g. tetrahydrofuran. Other metal compounds can be used, for example carbonates, e.g. sodium carbonate. For the preparation of ammonium salts, a stoichiometric amount or a slight excess of the corresponding organic amine in a suitable solvent, for

example a lower alkanol, e.g. methanol or ethanol, or an ether, e.g. diethyl ether, or another low-boiling solvent, is added to the compound of formula I. Acid addition salts of compounds of formula I are obtained in customary manner, for example by treatment with the corresponding mineral acid in an alcoholic or ethereal solvent. Inner salts can be formed, for example, by neutralizing salts, such as acid addition salts, to the isoelectric point, for example with weak bases, or by treatment with ion exchangers.

Salts can be converted in customary manner into the free compounds; metal and ammonium salts, for example, by treatment with suitable acids, and acid addition salts, for example, by treatment with a suitable basic agent. Salts can be transferred into other salts by stepwise preparing the free compound and then the other salt thereof as described above, by treating the salt with an excess of the corresponding salt-forming reagent and, if possible, crystallizing the desired salt from a suitable solvent, or by treating the salt with the corresponding ion exchange resin.

The starting materials used are known, or if novel, can be prepared according to the methods used in the references cited or as illustrated by the examples herein.

The above reactions are otherwise carried out according to standard methods, in the presence or absence of diluents, preferably such as are inert to the reagents and are solvents thereof, of catalysts, and/or of condensing or neutralization agents, and in air or under inert atmosphere, at low temperatures, room temperature or elevated temperatures, and at atmospheric or superatmospheric pressure.

The invention also comprises any modification of the above processes, wherein a compound resulting as an intermediate at any stage thereof is used as starting material and the remaining steps are carried out, or the process is discontinued at any stage thereof, or in which the starting material is formed under the reaction conditions or is used in the form of its salt or reactive derivative. In said processes of the invention those starting materials are advantageously selected which yield the above-described preferred embodiments of the invention.

The invention also relates to novel intermediates and processes for their manufacture.

Depending on the choice of starting materials and methods, the new compounds may be in the form of one isomer, tautomer or mixtures thereof, provided such are possible.

The compounds, including their salts, can also be obtained in the form of their hydrates, or include other solvents used for the crystallization.

The pharmaceutical compositions according to the invention are those suitable for enteral, such as oral or rectal, and parenteral administration to mammals, including man, for the alleviation and treatment of pain and inflammatory and arthritic diseases, such as osteoarthritis and rheumatoid arthritis, comprising an effective amount of a pharmacologically active compound of formula I or a pharmaceutically acceptable salt thereof, alone or in combination with one or more pharmaceutically acceptable carriers.

The pharmacologically active compounds of the invention are useful in the manufacture of pharmaceutical compositions containing an effective amount thereof in conjunction or admixture with excipients suitable for either enteral, parenteral or topical application. Preferred are tablets and gelatin capsules comprising the active ingredient together with (a) diluents, e.g. lactose, dextrose, sucrose, mannitol, sorbitol, cellulose and/or glycine; (b) lubricants, e.g. silica, talcum, stearic acid, its magnesium or calcium salt and/or polyethyleneglycol; for tablets also (c) binders, e.g. magnesium aluminum silicate, starch paste, gelatin, tragacanth, methyl cellulose, sodium carboxymethylcellulose and/or polyvinylpyrrolidone; if desired, (d) disintegrants, e.g. starches, agar, alginic acid or its sodium salt, or effervescent mixtures; and/or (e) absorbents, colorants, flavors and sweeteners. Injectable compositions are preferably aqueous isotonic solutions or suspensions. Suppositories or topical lotions are advantageously made from fatty emulsions or suspensions. They may be sterilized and/or contain adjuvants, such as preserving, stabilizing, wetting or emulsifying agents, solution promoters, salts for regulating the osmotic pressure and/or buffers. Said pharmaceutical compositions may also contain other therapeutically valuable substances. They are prepared according to conventional mixing, granulating or coating methods, respectively, and contain about 0.1 to 75%, preferably about 1 to 50%, of the active ingredient.

The invention relates also to a method of treating inflammatory and arthritic conditions in mammals, which comprises administering to said mammals a correspondingly effective amount of a compound of formula I or a pharmaceutically acceptable salt thereof.

The dosage of the active ingredient depends on the species, body weight, age and individual condition of the warm-blooded animal, on the disease to be treated and also on the mode of administration. A unit dosage for a mammal of about 50 to 70 kg may contain between about 10 to 200 mg of the active ingredient.

The following examples are intended to illustrate the invention and are not to be construed as being limitations thereon. Temperatures throughout are given in degrees Centigrade and all parts wherever given are parts by weight. If not otherwise stated, evaporations are carried out under reduced pressure, preferably

between about 20 mbar and 150 mbar.

## Example 1

p-Chloro-α-cyano-β-oxo-β-(2-thienyl)propionanilide

A suspension of 2.2 g of β-oxo-β-(2-thienyl)propionitrile in 50 ml of benzene is treated with 1.7 g of anhydrous triethylamine and then with a filtered solution of 2.3 g of p-chlorophenylisocyanate in benzene. An occurring mild exothermic reaction gives a dark red solution, which is let stand overnight. The benzene is evaporated, and the red oil taken up in methanol and poured into a solution of 5 ml of 5 N HCl in 300 ml of water. The brown solid is collected, washed with water, and triturated with ethanol to give the title product, m.p. 230-2°. Recrystallization from ethyl acetate gives pale yellow needles, m.p. 231-3°.

The starting material is prepared as follows:

A solution of 59.5 g of 2-acetylthiophene and 55 ml of ethyl formate in 150 ml of anhydrous diethyl ether is added with swirling to a suspension of sodium methoxide (freshly prepared from 9.2 g of sodium with anhydrous methanol; dried by distillation in vacuo on a steam cone) in 200 ml of dry ether. The sodium methoxide is consumed and the sodium salt of β-oxo-β-(2-thienyl)propanal precipitates. After standing at room temperature overnight, protected from moisture, the salt is collected and washed with dry ether.

To a solution of this sodium salt in 250 ml of methanol is added a solution of 28 g of hydroxylamine hydrochloride in 50 ml of water. The resulting red-brown solution is heated on a steam bath for 30 to 45 min, allowing about half of the methanol to evaporate. A solution of 16 g of sodium hydroxide in 200 ml of water is added, and heating on the steam bath resumed for 90 min. The solution is cooled, diluted with water, filtered through sintered glass, washed with ether and ethyl acetate, and acidified with 5 N hydrochloric acid. The product is collected, washed with water, air-dried, and triturated with ethanol-ether, giving β-oxo-β-(2-thienyl)propionitrile yellow crystals, m.p. 135-6° after recrystallization from ethanol.

## Example 2

General method for the preparation of α-cyano-β-oxo-β-(2-thienyl)propionanilides

Using essentially the procedure of Example 1, 2.1 g (0.014 mol) of β-oxo-β-(2-thienyl)propionitrile and 1.7 g (0.017 mol) of triethylamine in 50 ml of toluene, benzene or ethyl acetate are treated with 0.014 to 0.016 mol of the desired arylisocyanate. The resulting solution is let stand overnight, then evaporated, and the crude material taken up in methanol and treated with 0.1 N hydrochloric acid. The crude product is collected and purified by trituration with the appropriate indicated solvent or by dissolving in dilute NaOH solution, filtering, and reacidifying with 5 N HCl; and finally recrystallized from the appropriate indicated solvent to give the compounds listed in Table I.

Table 1

| α-Cyano-β-oxo-β-(2-thienyl)propionanilides | | | |
|---|---|---|---|
| Substituent of the anilide residue | Solvent for trituration | Solvent for recrystallization | m.p. |
| none | methanol | ethyl acetate | 194-5° |
| p-fluoro | ethanol/methanol | ethyl acetate | 221-3° |
| 3,4-dichloro | methanol | DMF/ethanol | 258-260° (dec.) |
| 3-chloro-4-fluoro | methanol | DMF/ethyl acetate | 255-6° |
| p-methoxy | NaOH/HCl | ethyl acetate | 193-5° |
| 2,4-dichloro | methanol | methanol/ethanol | 161-4° |
| 2,4-difluoro | NaOH/HCl or ethanol | methanol | 156-8° |
| m-trifluoromethyl | ethanol | methanol | 193-4° |
| m-chloro | methanol | acetone | 210-1° |
| m-fluoro | NaOH/HCl | methanol | 205-6° |
| 2,6-dimethyl | diethyl ether | ethanol | 124-5° |

## Example 3

α-Cyano-p-fluoro-β-oxo-β-(2-thienyl)propionanilide, alternative method

A solution of 1.0 g of ethyl α-cyano-β-oxo-β-(2-thienyl)propionate and 0.6 g of p-fluoroaniline in 50 ml of xylene is refluxed with azeotropic removal of low-boiling impurities for 165 min. On chilling the solution deposits crystals, which are collected and washed with ether. The product is identical with a sample prepared by the method of Example 2.

The starting material is prepared as follows:

A solution of 32 g of thiophene-2-carboxylic acid, 50 ml of thionyl chloride and 1 ml of DMF is refluxed on a steam cone for 15 min. The excess reagent is removed in vacuo, the residue taken up in chloroform and the solvent again removed by distillation in vacuo on a steam cone. The residual acid chloride (oil) is used without further purification.

50% sodium hydride (24 g) is washed with petroleum ether by decantation and suspended in 150 ml of dimethoxyethane (DME). Ethyl cyanoacetate (70 g) is added in portions with stirring and occasional ice bath cooling, and washed in with 25 ml additional DME. When the sodium hydride is nearly consumed, the thick, white suspension, with continued stirring, is treated with a solution of the thiophene-2-carboxylic acid chloride in 50 ml of DME from a dropping funnel, cooling with an ice bath as necessary to moderate the exothermic reaction. The orange suspension is stirred for several hours. Part of the solvent is removed by evaporation while warming gently on a steam cone. The material is then taken up in water, the aqueous solution washed once with ether and acidified with 5 N HCl. The crystals are collected, washed with water and air dried to give a tan solid, m.p. 71-74°. Recrystallization from petroleum ether or ethanol affords colorless crystals of ethyl α-cyano-β-oxo-β-(2-thienyl)propionate, m.p. 73-74°.

## Example 4

α-Cyano-β-oxo-N-(2-pyridyl)-β-(2-thienyl)propionamide

A solution of 6.0 g of ethyl α-cyano-β-oxo-β-(2-thienyl)propionate and 3.3 g of 2-aminopyridine in 250 ml of xylene is refluxed for 2 hrs. Upon chilling, a brown solid separates which is collected and triturated

successively with water and warm methanol to give above product, m.p. 236-7° (dec.). Recrystallization from dimethyl formamide (DMF) and methanol gives voluminous, colorless crystals; m.p. 241-2° (dec.).

## Example 5

α-Cyano-β-oxo-N-(2-thiazolyl)-β-(2-thienyl)propionamide

A solution of 6.0 g of ethyl α-cyano-α-oxo-β-(2-thienyl)propionate and 3.5 g of 2-aminothiazole in 250 ml of xylene is refluxed for 150 min. After chilling, crude product is collected and triturated, first with methanol, then with dilute hydrochloric acid. The obtained product is collected, washed with water and dried, giving a light-brown solid, m.p. 228-9° (dec.). The compound is recrystallized from DMF and methanol.

## Example 6

p-Chloro-2-cyano-3-oxo-5-(2-thienyl)-4-pentenanilide

A solution of 2.3 g of 3-oxo-5-(2-thienyl)-4-pentenenitrile in 40 ml of toluene and 1.5 g of anhydrous triethylamine is treated with 2.0 g of p-chlorophenyliso cyanate. After standing overnight, the toluene is evaporated on a steam cone. A filtered solution of the crude residue in methanol is added to a solution of 4 ml of 5 N HCl in 250 ml of water. The product is collected, washed with water, then triturated with methanol and dried to give yellow crystals, m.p. 255-9° (dec.). Recrystallization from DMF and methanol gives a pure sample, m.p. 260-262° (dec.).

The starting material 3-oxo-5-(2-thienyl)-4-pentenenitrile is prepared as follows:

50% sodium hydride (5.3 g) is washed with petroleum ether and suspended in 30 ml of DMF. A solution of 19 g of ethyl β-(2-thienyl)acrylate (prepared by esterification of the corresponding acid with ethanol and $H_2SO_4$) and 12 g of acetonitrile in some diethyl ether is added. The exothermic reaction is initiated by warming briefly and swirling on a steam cone, and allowed to proceed. After standing overnight, the solidified, brown mixture is diluted with dry diethyl ether and the crude sodium salt of the β-ketonitrile collected. An aqueous solution of this salt combined with an aqueous extract of the ether solution are treated with Norit and acidified with 10 ml of glacial acetic acid. After chilling, the product is collected, washed with water, dried, and triturated with ether and a small amount of ethanol, to give yellow crystals, m.p. 92-94°. Recrystallization from diethyl ether and petroleum ether raises this m.p. to 93-95°.

## Example 7

2-Cyano-p-fluoro-3-oxo-5-(2-thienyl)-4-pentenanilide

A solution of 2.1 g of 3-oxo-5-(2-thienyl)-4-pentenenitrile in 40 ml of toluene and 1.4 g of anhydrous triethylamine is treated with 1.7 g of p-fluorophenylisocyanate. The reaction mixture is worked up as described in Example 6. The crude product obtained after trituration with methanol is recrystallized from DMF and methanol, m.p. 244-6°.

## Example 8

3-Chloro-α-cyano-4-fluoro-β-(2-furyl)-β-oxopropionanilide

A solution of 1.9 g (0.014 mol) of β-(2-furyl)-β-oxopropionitrile and 1.7 g of anhydrous triethylamine in 40 ml of toluene is treated with 2.5 g (0.0145 mol) of 3-chloro-4-fluorophenylisocyanate, with stirring, and the reaction mixture let stand overnight. The toluene is evaporated, the residue taken up in methanol, and added to dilute HCl (4 ml of 5 N HCl in 250 ml of water). The product is collected, washed with water, pressed dry, then triturated with warm methanol. The compound is collected as a tan solid, m.p. 250-1° (dec.). Recrystallization from DMF and ethyl acetate gives colorless crystals, m.p. 261-2° (dec.).

The starting material β-(2-furyl)-β-oxopropionitrile is prepared as follows:

To a suspension of 16.8 g of 50% sodium hydride in 25 ml each of DMF and dimethoxyethane is added a solution of 56 g of ethyl α-furoate and 25 g of acetonitrile in 25 ml of diethyl ether and 25 ml of DMF. The suspension is warmed on a steam cone with swirling until exothermic reaction with consumption of the sodium hydride and effervescence commences (ca. 5 min.), then swirled and occasionally cooled briefly in an ice bath to control the rate of reaction; as it nears completion, 8 ml of additional acetonitrile is added. When all the sodium hydride is consumed, the suspension is let stand overnight. Dry ether is added, and the red-brown sodium salt is collected; this is treated with 25 ml of glacial acetic acid and then with water. The product is collected, washed with water, and air dried. The nitrile, a tan solid, m.p. 78-80°, is recrystallized from ethanol to give yellow flakes, m.p. 79-80°.

## Example 9

### General method for the preparation of α-cyano-β-(2-furyl)-β-oxopropionanilides

Following essentially the procedure of Example 8, 1.9 g (0.014 mol) of β-(2-furyl)-β-oxopropionitrile and 1.7 g (0.017 mol) of triethylamine in 40 ml of toluene are treated with 0.014-0.016 mol of the desired arylisocyanate. The solution, after standing overnight, is evaporated, taken up in methanol and acidified with 0.1 N hydrochloric acid. The crude product is collected and purified, if required, by trituration with the appropriate indicated solvent and by recrystallization from the appropriate indicated solvent.

Table 2

| α-Cyano-β-(2-furyl)-β-oxopropionanilides | | | |
|---|---|---|---|
| Substituent of the anilide residue | Solvent for trituration | Solvent for recrystallization | m.p. |
| none | - | ethanol | 184-5° |
| p-fluoro | methanol | ethyl acetate | 240-1° |
| p-chloro | methanol | ethyl acetate | 237-8° |
| 3,4-dichloro | methanol | dimethyl formamide | 259-261° |
| p-methoxy | methanol | ethyl acetate | 193-5° |
| 2,4-dichloro | methanol | ethyl acetate | 190-1° |
| 2,4-difluoro | ethanol | methanol | 165-6° |
| m-trifluoromethyl | methanol | methanol | 186-7° |
| m-chloro | - | ethyl acetate | 219-220° |
| 2,6-dimethyl | - | ethanol | 132-3° |

## Example 10

### β-(5-Bromo-2-furyl)-p-chloro-α-cyano-β-oxopropionanilide

A solution of 1.7 g of β-(5-bromo-2-furyl)-β-oxopropionitrile and 0.9 g triethylamine in 60 ml of toluene is treated with 1.3 g of p-chlorophenylisocyanate. A mildly exothermic reaction takes place, and the mixture is let stand overnight. The toluene is evaporated, and the residue is taken up in methanol and added to a solution of 2 ml of 5 N HCl in 200 ml of water. The product is collected, washed with water, air dried, and triturated with warm methanol to give crystals, m.p. 238-241° (dec.). Recrystallization from DMF and ethyl acetate gives yellow crystals, m.p. 249-251° (dec.).

The starting material β-(5-bromo-2-furyl)-β-oxopropionitrile, m.p. 158-9° (from ethanol) is essentially prepared as described for β-(2-furyl)-β-oxopropionitrile in Example 8, using 2.4 g of 50% sodium hydride, 11 g of ethyl 5-bromo-2-furoate (Ann. Chem. 232, 51) and 4.1 g of acetonitrile.

## Example 11

### α-Cyano-p-fluoro-β-oxo-β-(3-pyridyl)propionanilide

A suspension of 4.7 g of the sodium salt of β-oxo-β-(3-pyridyl)propionitrile in 35 ml of dimethylsulfoxide is treated with 4.5 g of p-fluorophenylisocyanate. Following the mild exothermic reaction, the mixture is let stand overnight. The mixture is treated with water (ca. 400 ml) and 5 ml of 10% NaOH solution, and the solution is filtered and carefully acidified with 5 N HCl. The product is collected, washed with water, and air dried to give yellow crystals, m.p. 231-2° (dec.). The compound is recrystallized from DMF and ethanol.

The starting material β-oxo-β-(3-pyridyl)propionitrile is prepared as follows:

Dry sodium ethoxide, prepared from 6.2 g of sodium, is suspended in 400 ml of dry toluene. A solution of 45 g of ethyl nicotinate and 24 g of acetonitrile in 200 ml of toluene is added, and the suspension is refluxed with stirring for 3 hrs. The thick suspension is cooled, diluted with anhydrous diethyl ether, and the sodium salt of the β-ketonitrile is collected, washed with anhydrous diethyl ether, and dried. This material, which displays a strong CN peak at 4.59 µm in the infrared spectrum, is used without further purification.

Employing the same procedure, the following analogs are prepared using the appropriate isocyanate:

Table 3

| α-Cyano-β-oxy-β-(3-pyridyl)propionanilide | | |
|---|---|---|
| Substituent of the anilide residue | Solvent for recrystallization | m.p. |
| none | DMF/ethanol | 229-230° (dec.) |
| p-chloro | DMF/ethanol | 247-8° (dec.) |
| 3,4-dichloro | DMF/ethanol | 237-9° (dec.) |
| 3-chloro-4-fluoro | DMF/methanol | 232-3° (dec.) |

## Example 12

### p-Chloro-α-cyano-β-oxo-β-(4-pyridyl)propionanilide

A solution of 3.2 g of the sodium salt of β-oxo-β-(4-pyridyl)propionitrile in 10 ml dimethylsulfoxide is treated with 3.0 g of p-chlorophenylisocyanate. After the occurring exothermic reaction, the solution is let stand overnight. Water (ca. 300 ml) and 10 ml of 10% NaOH solution are added, and after stirring the suspension is filtered. The filtrate is carefully acidified with 5 N HCl, the product is collected, washed with water, dried, and triturated with methanol to give yellow-orange crystals; m.p. 244-5° (dec.). Recrystallization from DMF and methanol raises the m.p. to 248-9° (dec.).

The starting material β-oxo-β-(4-pyridyl)propionitrile is prepared as follows:

To a suspension of 4.8 g of 50% sodium hydride in 10 ml of DMF is adced a solution of 16 g of ethyl isonicotinate and 8.2 g of acetonitrile in 10 ml of DMF. The reaction is initiated by warming on a steam cone, then allowed to proceed, with swirling and occasional cooling as necessary to moderate the vigorously exothermic effect. Additional acetonitrile (5 ml) and DMF (10 ml) are added. After standing 4 hrs, the thick suspension is diluted with anhydrous diethyl ether. The sodium salt of the β-ketonitrile is collected, washed with diethyl ether, and dried. The material of m.p. 287-297° (dec.) displays the expected CN absorption in the infrared spectrum (4.59 µm) and is used without further purification.

## Example 13

α-Cyano-p-fluoro-β-oxo-β-(4-pyridyl)propionanilide

A solution of 4.2 g of the sodium salt of β-oxo-β-(4-pyridyl)propionitrile in dimethylsulfoxide is acylated with 3.0 g of p-fluorophenylisocyanate, following the procedure of Example 12. The title compound is obtained as orange crystals, m.p. 228-9°, and is recrystallized from DMF and ethanol.

## Example 14

α-Cyano-p-fluoro-3-(1,5-dimethyl-3-pyrazolyl)-β-oxopropionanilide

A solution of 2.6 g of β-(1,5-dimethyl-3-pyrazolyl)-β-oxopropionitrile and 1.8 g of triethylamine in 20 ml of dimethoxyethane is treated with 2.4 g of p-fluorophenylisocyanate. After standing overnight the crystalline, crude mixture is treated with dilute HCl and the product collected, washed with water, dried, and triturated with methanol and ethyl acetate to give colorless crystals, m.p. 235-240°. After recrystallization from ethyl acetate, the compound melts at 239-241°.

The starting material is prepared as follows:

58 g of acetone and 146 g of ethyl oxalate are condensed with a solution of 26 g of sodium in 500 ml of reagent methanol. The obtained sodium salt of methyl 2,4-dioxovalerate is washed with ethanol and dried, m.p. 260-1° (dec.).

Following the method of v. Auwers and Hollmann, Ber. 59, 605, 1282 (1926), an aqueous solution (100 ml) of 20 g of the sodium salt of methyl 2,4-dioxovalerate is treated with a solution of 6.4 ml of methylhydrazine in aqueous sulfuric acid prepared from 12.5 g of cold conc. $H_2SO_4$ and 20 g of ice. After stirring for 1 hr, the solution is carefully made alkaline with sodium hydroxide solution. The products are extracted with diethyl ether, and the etheral solution dried with sodium sulfate and evaporated. On trituration with cold diethyl ether, crystals of methyl 1,5-dimethyl-3-pyrazolylcarboxylate, m.p. 72-5°, are obtained. Recrystallization from diethyl ether raises the m.p. to 75-7°. The etheral filtrate from the trituration is evaporated, and the residual oil distilled in vacuo, affording methyl 1,3-dimethyl-5-pyrazolylcarboxylate, b.p. 100-102°/12 mbar.

A solution of 13.2 g of methyl 1,5-dimethyl-3-pyrazolylcarboxylate in 20 ml of acetonitrile is added to a suspension of 6.7 g of 50% sodium hydride (washed oil-free by decantation with petroleum ether) in 25 ml of DMF. The reaction is initiated by warming briefly on a steam cone, with swirling. After standing overnight, the solidified mixture is treated with water, the solution filtered, and acidified to pH 6 with 5 N HCl. After chilling, the crystalline product is collected, washed with cold water, and dried. Recrystallization from ethanol gives colorless crystals of β-(1,5-dimethyl-3-pyrazolyl)-β-oxopropionitrile, m.p. 114-6°.

## Example 15

α-Cyano-β-(1,5-dimethyl-3-pyrazolyl)-β-oxopropionanilide

A solution of 1.3 g of β-(1,5-dimethyl-3-pyrazolyl)-β-oxopropionitrile and 0.9 g of triethylamine in 30 ml of toluene is treated with 1.05 g of phenylisocyanate. After standing overnight, evaporation, and treatment with a solution of 3 ml of 5 N HCl in 250 ml of water, the crude solid is purified by dissolving in dilute NaOH solution, filtering, reacidifying with 5 N HCl to give, after collecting, washing with water and air drying, crystals, m.p. 219-221°. Recrystallization from ethanol and ethyl acetate, and treatment with Norit gives colorless crystals, m.p. 233-5°.

## Example 16

α-Cyano-β-(1,3-dimethyl-5-pyrazolyl)-3-oxopropionanilide

A suspension of 1.95 g of β-(1,3-dimethyl-5-pyrazolyl)-β-oxopropionitrile and 1.5 g of triethylamine in 40 ml of warm toluene is treated with 1.9 g of phenylisocyanate. After the mild exothermic reaction and standing overnight, the red-orange suspension is diluted with dry diethyl ether, and the solid collected. This material is dissolved in a solution of 10 ml of 10% NaOH and 200 ml of water, with the aid of a little methanol. The by-product (diphenylurea) is filtered off, and the filtrate acidified with 6 N HCl. The product is collected, washed with water, and dried; colorless crystals, m.p. 201-3°. This product may be recrystallized from methanol.

The starting material is prepared as follows:

2.4 g of 50% sodium hydride are washed with petroleum ether, suspended in 15 ml of DMF, and mixed with 3.7 g of methyl 1,3-dimethyl-5-pyrazolylcarboxylate of Example 14 in 10 ml of acetonitrile. The procedure described for the corresponding 1,5-dimethyl-3-pyrazolyl isomer in Example 14 is followed, which gives β-(1,3-dimethyl-5-pyrazolyl)-β-oxopropionitrile, m.p. 140-1°. Recrystallization from water gives crystals with m.p. 141-2°.

## Example 17

α-Cyano-β-(5-methyl-3-isoxazolyl)-β-oxopropionanilide

A suspension of 3.8 g of the sodium salt of P-(5-methyl-3-isoxazolyl)-β-oxopropionitrile in 25 ml of DMF is treated with 3.7 g of phenylisocyanate. After the exothermic, somewhat effervescent reaction, the mixture is let stand several hours. Water is added, the mixture filtered to remove the by-product diphenylurea, and the filtrate acidified with 3 ml of 6 N HCl. The product is collected, washed with water, dried, and triturated with ethanol, to give a tan solid, m.p. 201-4°. Recrystallization from ethyl acetate gives pale yellowish crystals, m.p. 205-7°.

The starting material is prepared as follows:

20 g of the sodium salt of methyl 2,4-dioxovalerate in 100 ml of methanol are neutralized with 12 ml of conc. HCl. 10.5 g of hydroxylamine hydrochloride in 10 ml of water are added, and the mixture refluxed for 90 min. The methanol is removed by distillation on a steam cone with an aspirator. Water is added, and colorless crystals of methyl 5-methylisoxazolyl-3-carboxylate, m.p. 89-91°, are collected. Sodium ethoxide, prepared from 1.5 g of sodium, is dried and suspended in 60 ml of dry toluene. A solution of 9.2 g of methyl 5-methylisoxazolyl-3-carboxylate and 7 g of acetonitrile in 40 ml of toluene is added, and the suspension refluxed for 2 hrs. After evaporation of about half of the toluene, dry ether is added and the sodium salt of β-(5-methyl-3-isoxazolyl)-β-oxopropionitrile collected, washed with diethyl ether, and dried. The product is a yellow-orange solid, m.p. 205-7° (dec.), and is used without further purification.

## Example 18

α-Cyano-p-fluoro-β-(5-methyl-3-isoxazolyl)-β-oxopropionanilide

A suspension of 5.3 g of the sodium salt of β-(5-methyl-3-isoxazolyl)-β-oxopropionitrile in 20 ml of DMF is allowed to react with 6.0 g of p-fluorophenylisocyanate. After several hours the reaction mixture is worked up as described in Example 17. The resulting beige solid, m.p. 220-5°, is triturated with warm ethanol and affords crystals, m.p. 228-231° (dec.). Recrystallization from DMF and ethyl acetate gives a pure sample; shiny, cream-colored flakes, m.p. 235-7° (dec.).

## Example 19

### α-Cyano-β-(1-methyl-5-imidazolyl)-β-oxopropionanilide

A suspension of 5.8 g of the sodium salt of β-(1-methyl-5-imidazolyl)-β-oxopropionitrile in 25 ml of DMF is treated with 7.4 g of phenylisocyanate, in portions. An exothermic, vigorously effervescent reaction occurs, giving a red-brown solution. After standing overnight, this is diluted with water, and the by-product diphenylurea filtered off. The filtrate is carefully neutralized with 5 N HCl. The product is collected, washed with water, and dried; yellow-orange solid, m.p. 225-7° (dec.). Recrystallization from methanol gives a pure sample, pink crystals, m.p. 239-241° (dec).

The starting material is prepared as follows:

Ethyl 1-methylimidazolyl-5-carboxylate, b.p. 133-4°/8 mbar, is prepared from sarcosine ethyl ester by successive N- and C-formylation, closure with thiocyanic acid, and oxidative desulfurization with nitric acid, as described by R. G. Jones, J. Am. Chem. Soc. 71, 644 (1949).

3.1 g of 50% sodium hydride are washed by decantation with petroleum ether and suspended in 20 ml of DMF. A solution of 7.7 g of ethyl 1-methylimidazolyl-5-carboxylate and 20 ml of acetonitrile is added. The reaction is initiated by warming on a steam cone with swirling, and allowed to proceed exothermically. After standing overnight, the suspension is diluted with dry ethyl ether and the solid collected, washed with diethyl ether and dried. The resulting sodium salt of β-(1-methyl-5-imidazolyl)-β-oxopropionitrile, tan solid, m.p. ca. 180° (dec.), is used without further purification. A sample of the sodium salt is treated with glacial acetic acid, the free imidazolyl β-ketonitrile extracted with acetone, and converted to the corresponding hydrochloride for characterization; light orange crystals from ethanol and methanol, m.p. 210-2° (dec.).

## Example 20

### α-Cyano-p-fluoro-β-(1-methyl-5-imidazolyl)-β-oxopropionanilide

A suspension of 4.3 g of the sodium salt of β-(1-methyl-5-imidazolyl)-β-oxopropionitrile in 25 ml of DMF is treated with 5.5 g of p-fluorophenylisocyanate, in portions. Following the exothermic and somewhat effervescent reaction, the brown mixture is let stand overnight. Water is added, and the by-product diarylurea removed by filtration. The filtrate is neutralized carefully by adding 2.6 ml of 6 N HCl and 1 ml of glacial acetic acid. The product is collected, washed, and dried; yellowish crystals, m.p. 228-230° (dec.). Recrystallization from DMF and ethanol gives nearly colorless crystals, m.p. 235-6° (dec.).

## Example 21

### p-Chloro-α-cyano-β-(1-methyl-5-imidazolyl)-β-oxopropionanilide

A suspension of 4.8 g of the sodium salt of β-(1-methyl-5-imidazolyl)-β-oxopropionitrile in 25 ml of DMF is mixed with 6.6 g of p-chlorophenylisocyanate to give a brown suspension which, after standing overnight, is treated with water and 2 ml of 10% NaOH, filtered, and the filtrate neutralized with glacial acetic acid. The product is collected, washed with water, and dried; yellow crystals, m.p. 239-241° (dec.). Recrystallization from DMF and ethanol gives pale yellow, voluminous crystals, m.p. 245-6° (dec.).

Example 22

α-Cyano-β-(1-methyl-2-imidazolyl)-β-oxopropionanilide

A solution of 2.4 g of the sodium salt of β-(1-methyl-2-imidazolyl)-β-oxopropionitrile in 15 ml DMF is treated with 2.0 g of phenylisocyanate. After the mild exothermic reaction, the yellow solution is let stand overnight. Water is added, the solution filtered, and the filtrate carefully acidified to pH 6 with 3 ml of 6 N HCl. The product is collected, washed, and dried; yellow crystals, m.p. 224-5° (dec.). Recrystallization from methanol gives pale yellow crystals, m.p. 227-9° (dec.).

The starting material is prepared as follows:

Following C.G. Begg et al., Australian J. Chem., 26, 415 (1973), ethyl 1-methylimidazolyl-2-carboxylate is obtained by treatment of N-methylimidazole with ethyl chloroformate in acetonitrile, followed by addition of triethylamine, filtration, evaporation, and distillation in vacuo; oil, b.p. 130-20/4.5 mbar.

Sodium ethoxide is prepared from 2.1 g of sodium and ethanol, dried in vacuo, and suspended in 200 ml of dry toluene. To this suspension is added a solution of 13.0 g of ethyl 1-methylimidazolyl-2-carboxylate and 7 g of acetonitrile in 50 ml of toluene. The suspension is refluxed with stirring for 150 min. The red suspension is diluted with 100 ml of dry ethyl ether and the sodium salt of β-(1-methyl-2-imidazolyl-β-oxopropionitrile collected, washed with ethyl ether and dried; yellow-orange solid, m.p. 217-226°. A sample of the sodium salt is treated with glacial acetic acid, and the free β-ketonitrile triturated with ether and acetone and recrystallized from ethanol; needles, m.p. 109-111°.

Example 23

α-Cyano-p-fluoro-β-(1-methyl-2-imidazolyl)-β-oxopropionanilide

The compound is obtained by reaction of the sodium salt of β-(1-methyl-2-imidazolyl)-β-oxopropionitrile with p-fluorophenylisocyanate following the procedure of Example 22; solid, m.p. 241-2° (dec.). Recrystallization from ethanol and DMF gives colorless crystals, m.p. 243-4° (dec.).

Example 24

p-Chloro-α-cyano-β-(1-methyl-2-imidazolyl)-β-oxopropionanilide

The compound is obtained by reaction of the sodium salt of β-(1-methyl-2-imidazolyl)-β-oxopropionitrile with p-chlorophenylisocyanate following the procedure of Example 22; solid, m.p. 235-6° (dec.). Recrystallization from ethanol and methanol gives crystals, m.p. 238-9° (dec.).

Example 25

α-Cyano-β-(2,4-dimethyl-5-thiazolyl)-β-oxopropionanilide

1.55 g 50% sodium hydride is washed with petroleum ether, suspended in 10 ml of DMF, and treated in portions with 5.3 g of cyanoacetanilide in 15 ml of dimethoxyethane (DME). When the sodium hydroxide is consumed, a solution of 2,4-dimethylthiazolyl-5-carbonyl chloride in 10 ml of DME is added with stirring. Moderately exothermic reaction gives a purplish mixture. After standing overnight, part of the solvent is evaporated, the material taken up in water, filtered to remove unreacted cyanoacetanilide, and the red filtrate acidified with 5 ml of 6 N HCl. The product is collected, washed with water, dried and triturated with

cold ethanol, giving tan crystals, m.p. 178-180°. Recrystallization from ethanol raises the m.p. to 180-181°.

The starting material is prepared as follows:

12.0 g of thioacetamide and 25 g of ethyl α-chloroacetoacetate in 25 ml of ethanol are induced to react by warming on a steam cone. Treatment of the resulting hydrochloride with water and $Na_2CO_3$ affords ethyl 2,4-dimethylthiazolyl-5-carboxylate, m.p. 48-50°. Hydrolysis of this ester (6.3 g) with 10% NaOH solution (25 ml) by heating on a steam cone for 5 min and allowing to stand for 30 min; filtration, and acidification with 6 N HCl (11 ml), gives the corresponding acid, which is collected, washed with water, and dried; colorless crystals of 2,4-dimethylthiazolyl-5-carboxylic acid, m.p. 233-4° (dec.).

Following W.R. Boon, J. Chem. Soc. 601 (1945), a suspension of 2.5 g of this thiazole acid in 50 ml of dry diethyl ether and 1.3 g of pyridine are chilled and treated slowly with a solution of 1.9 g of thionyl chloride in 10 ml of diethyl ether. After stirring for 90 min at room temperature, the suspension is filtered, and the filtrate evaporated in vacuo with very gentle warming, to give crude 2,4-dimethylthiazolyl-5-carbonyl chloride which is used directly in the next step.

## Example 26

α-Cyano-p-fluoro-β-(2,4-dimethyl-5-thiazolyl)-β-oxopropionanilide

The compound is obtained by reaction of 2,4-dimethylthiazolyl-5-carbonyl chloride with α-cyano-p-fluoroacetanilide following the procedure of Example 25; solid, m.p 196-8°. Recrystallization from methanol and ethanol gives crystals, m.p. 199-200°.

## Example 27

p-Chloro-α-cyano-β-(2,4-dimethyl-5-thiazolyl)-β-oxopropionanilide

The compound is obtained by reaction of 2,4-dimethylthiazolyl-5-carbonyl chloride with p-chloro-α-cyanoacetanilide following the procedure of Example 25; solid, m.p. 203-5°. Recrystallization from ethyl acetate gives crystals, m.p 208-9°.

## Example 28

α-Cyano-β-oxo-β-(2-phenyl-4-thiazolyl)propionanilide

A solution of 2.5 g of β-oxo-β-(2-phenyl-4-thiazolyl)propionitrile and 1.5 g triethylamine in 40 ml of toluene is treated with 1.6 g of phenylisocyanate. After standing overnight, the oily mixture is evaporated on a steam cone to remove toluene, and the residue dissolved in a little methanol and treated with a solution of 5 ml of 6 N HCl in 300 ml of water. The product is extracted with ethyl acetate, the organic solution washed with water, dried with magnesium sulfate and evaporated, and the crude product triturated with ethyl ether, giving crystals, m.p. 192-6°. Recrystallization from ethyl acetate gives colorless flakes, m.p. 198-200°.

The starting material is prepared as follows:

7 g of thiobenzamide in 100 ml of ethanol are mixed with 10 g of ethyl bromopyruvate. The solution is heated on a steam cone for 1 hr, evaporated, treated with water, and the oil extracted with ether, washed with water, dried with magnesium sulfate and evaporated to give ethyl 2-phenylthiazolyl-4-carboxylate.

4.8 g of 50% sodium hydride are washed with petroleum ether and suspended in 25 ml of dimethoxyethane. A solution of 11.8 g of ethyl 2-phenylthiazolyl-4-carboxylate in 25 ml of acetonitrile is added, and the mixture is heated on a steam cone with swirling, as needed to promote reaction, for 30 min. The solvent is evaporated, the residue dissolved in water, the solution filtered clear and acidified with 6 N HCl. The product is collected, washed with water, dried, and triturated with ethanol to give β-oxo-β-(2-phenyl-4-thiazolyl)propionitrile as brownish crystals; m.p. 141-7°. Recrystallization from methanol and ethyl acetate affords crystals, m.p. 149-152°. Further recrystallization from ethanol or from ethyl acetate raises the m.p. to 151-3°.

## Example 29

α-Cyano-p-fluoro-β-oxo-β-(2-phenyl-4-thiazolyl)propionanilide

The compound is obtained by reaction of β-oxo-β-(2-phenyl-4-thiazolyl)propionitrile with p-fluorophenylisocyanate following the procedure of Example 28. After trituration with ethanol and methanol, the product of m.p. 214-6° is collected. Recrystallization from ethyl acetate raises the m.p. to 215-6°.

## Example 30

p-Chloro-α-cyano-β-oxo-β-(2-phenyl-4-thiazolyl)propionanilide

The compound is obtained by reaction of β-oxo-β-(2-phenyl-4-thiazolyl)propionitrile with p-chlorophenylisocyanate following the procedure of Example 28. After trituration with ethanol and methanol, the product of m.p. 222-5° is collected. Recrystallization from ethyl acetate raises the m.p. to 224-6°.

## Example 31

p-Chloro-α-cyano-β-oxo-β-phenylpropionanilide

A solution of 3.2 g of benzoyloacetonitrile (β-oxo-β-phenylpropionitrile) in 50 ml of benzene and 2.5 g of anhydrous triethylamine is treated with a solution of 3.4 g of p-chlorophenylisocyanate in 20 ml of benzene. Following the mild exothermic reaction the solution is let stand overnight. The benzene is evaporated, and the residue dissolved in a little methanol is added to 15 ml of 5 N HCl in 300 ml of water. The product is collected, washed with water, dried, and triturated with methanol to give colorless crystals, m.p. 242-4°. Recrystallization from DMF raises the m.p. to 246-8°.

Employing the same procedure, the following analogs are prepared using the appropriate isocyanates:

Table 4

| Halo-substituted α-cyano-β-oxo-β-phenylpropionanilides | | |
|---|---|---|
| Substituent of the anilide residue | Solvent for recrystallization | m.p. |
| p-fluoro | ethyl acetate | 229-231° |
| 3,4-dichloro | ethyl acetate | 230-1° |
| 3-chloro-4-fluoro | ethyl acetate | 225-6° |
| 2,4-difluoro | methanol | 159-161° |
| 2,3-dichloro | ethyl acetate | 185-6° |

Example 32

α-Cyano-2,4-difluoro-β-(p-methoxyphenyl)-β-oxopropionanilide

A suspension of 2.1 g of p-methoxybenzoylacetonitrile (β-p-methoxyphenyl-β-oxopropionitrile) in 50 ml of toluene and 1.5 g of anhydrous triethylamine is treated with 2.0 g of 2,4-difluorophenylisocyanate. The mixture, after warming for a few minutes on a steam cone, is let stand overnight. The toluene is evaporated, and a solution of the residue in a little methanol is treated with a solution of 4 ml of 5 N HCl in 250 ml of water. The collected, crude product is triturated with cold ethanol and again filtered giving crystals of m.p. 163-5°. Recystallization from methanol gives colorless crystals, m.p. 163-5°.

The starting material is prepared as follows:

6.2 g of sodium are dissolved in reagent methanol, the excess methanol removed by distillation with an aspirator, and the sodium methoxide suspended in 200 ml of anhydrous ether. To this suspension is added a solution of 45 g of p-methoxyacetophenone and 30 ml of ethyl formate in 100 ml of anhydrous diethyl ether, with swirling. After standing several hours the suspension of the sodium salt of β-(p-methoxyphenyl)-β-oxopropanal is filtered with the aid of more anhydrous ether and the salt washed with ether and dried.

To a solution of 30 g of above sodium salt of β-(p-methoxyphenyl)-β-oxopropanal and 6 g of NaOH in 250 ml of water is added a solution of 10.5 g of hydroxylamine hydrochloride in 50 ml of water. The solution is heated on a steam cone for 4 hrs. The cooled solution is diluted with water to 500 ml, washed with ether and ethyl acetate and neutralized with 10 ml of glacial acetic acid. The precipitated product is collected, washed with water, air dried and recrystallized from ethanol; yellow crystals of β-(p-methoxyphenyl)-β-oxopropionitrile, m.p. 130-2°.

Employing the same procedure, the following analogs are prepared using the appropriate isocyanates and the indicated solvents for trituration and for recrystallization:

Table 5

| α-Cyano-β-(p-methoxyphenyl)-β-oxopropionanilides | | | |
|---|---|---|---|
| Substituent of the anilide residue | Solvent for trituration | Solvent for recrystallization | m.p. |
| none | methanol/ethanol | ethyl acetate | 187-8° |
| p-fluoro | ethanol | ethyl acetate | 227-9° |
| p-chloro | methanol | DMF | 246-8° |
| 3,4-dichloro | methanol | ethyl acetate | 218-220° |

Example 33

p-Chloro-β-(chlorophenyl)-α-cyano-β-oxopropionanilide

To a solution of 2.2 g of p-chlorobenzoylacetonitrile (β-p-chlorophenyl-β-oxopropionitrile) in 30 ml of toluene and 1.6 g of anhydrous triethylamine is added a solution of 2.3 g of p-chlorophenylisocyanate in 10 ml of toluene. Following the mild exothermic reaction, on standing overnight the triethylammonium salt of the product separates as crystals. This is collected, washed with toluene and ether, and dried; m.p. 120-121°. Treatment of this solid with dilute HCl (3 ml of 5 N HCl in 250 ml of water) gives a tan solid, which is collected, washed with water, and dried. The product is purified by dissolving in dilute NaOH and reacidifying the filtered solution with 5 N HCl; the precipitate is collected, washed with water, and dried to give colorless crystals, m.p. 228-230°. Recrystallization from ethyl acetate raises the m.p. to 231-3°.

The starting material is prepared as follows:

Following the procedure of Example 32 for the formation of the corresponding β-(p-methoxyphenyl)-β-oxopropionitrile, 34 g of p-chloroacetophenone are formylated with 25 ml of ethyl formate in the presence of sodium methoxide prepared from 4.6 g of sodium. The obtained sodium salt of β-(p-chlorophenyl)-β-oxopropanal is collected, washed with anhydrous diethyl ether, and dried. 12 g of this sodium salt, 4.3 g of hydroxylamine hydrochloride and 2.5 g of NaOH in 300 ml of water are heated on a steam cone for 3 hrs. The cooled solution is filtered, acidified with 5 ml of glacial acetic acid and 2 ml of 5 N HCl, and the precipitate collected, washed with water and dried. Recrystallization from diethyl ether gives β-(p-chlorophenyl)-β-oxopropionitrile; orange needles, m.p. 128-130°.

## Example 34

### Capsules

1,000 capsules, each containing 25 mg of the active ingredient, are prepared using the following formula:
3,4-Dichloro-α-cyano-β-oxo-β-(2-thienyl)propionanilide: 25.0 g
Lactose: 207.0 g
Modified starch: 80.0 g
Magnesium stearate: 3.0 g

All the powders are passed through a screen with openings of 0.6 mm. Then the drug substance is placed in a suitable mixer and mixed first with the magnesium stearate, then with the lactose and starch until homogeneous. No. 2 hard gelatin capsules are filled with 315 mg of said mixture each, using a capsule filling machine.

Analogously, capsules are prepared containing 10-200 mg of the other compounds disclosed and illustrated herein.

## Example 35

### Tablets

10,000 tablets, each containing 100 mg of the active ingredient, are prepared using the following formula
α-Cyano-m-trifluoromethyl-β-oxo-β-(2-thienyl)propionanilide: 1000 g
Lactose: 2535 g
Corn starch: 125 g
Polyethylene glycol 6000: 150 g
Talcum powder: 150 g
Magnesium stearate: 40 g
Purified water: q.s.

All the powders are passed through a screen with openings of 0.6 mm. Then the drug substance, lactose, talcum, magnesium stearate and half of the starch are mixed in a suitable mixer. The other half of the starch is suspended in 65 ml of water and the suspension added to the boiling solution of the polyethylene glycol in 260 ml of water. The paste formed is added to the powders, which are granulated, if necessary, with an additional amount of water. The granulate is dried overnight at 35°, broken on a screen with 1.2 mm openings and compressed into tablets, using concave punches uppers bisected.

Analogously, tablets are prepared containing 10-200 mg of one of the other compounds illustrated by the previous examples.

**Claims**

1. A compound of formula

$$\begin{array}{c} \quad\quad\quad CN \\ \quad\quad\quad | \\ A\text{-}CO\text{-}\ CH\ \text{-}CONH\text{-}B \end{array} \quad (I),$$

wherein A is selected from the group consisting of five- and six-membered unsubstituted or substituted aromatic heterocyclyl with the exception of pyrrolyl, five- and six-membered aromatic heterocyclyl-vinyl, and unsubstituted and substituted phenyl, and wherein B is unsubstituted or substituted phenyl, pyridyl or thiazolyl, with the proviso that when A is unsubstituted phenyl B does not represent unsubstituted phenyl, methylphenyl or methoxyphenyl; or an enol tautomer, lower alkyl enol ether or salt thereof.

2. A compound as claimed in claim 1 of formula I wherein
A is selected from the group consisting of five membered aromatic heterocyclyl comprising one oxygen atom, one sulfur atom, or two heteroatoms wherein one is nitrogen and the other one is nitrogen, oxygen or sulfur, and wherein the aromatic heterocycle is unsubstituted or substituted at carbon atoms by one, two or three residues selected from lower alkyl, phenyl, furyl, thienyl and halogen, and at a nitrogen atom by lower alkyl; six-membered aromatic heterocyclyl comprising one or two nitrogen atoms, unsubstituted or substituted at carbon atoms by lower alkyl or phenyl; furylvinyl; thienylvinyl; and phenyl substituted by one or two residues selected from lower alkyl, phenyl, hydroxy, lower alkoxy, halogen and trifluoromethyl; and
B is selected from the group consisting of phenyl; phenyl substituted by one or two residues selected from lower alkyl, phenyl, hydroxy, lower alkoxy, halogen and trifluoromethyl; pyridyl; and thiazolyl;
or an enol tautomer, lower alkyl enol ether or salt thereof.

3. A compound as claimed in claim 1 of formula I wherein A is phenyl and B is phenyl substituted by one or two residues selected from halogen and trifluoromethyl, or an enol tautomer, lower alkyl enol ether or salt thereof.

4. A compound as claimed in claim 1 of formula I wherein A is five- or six-membered aromatic heterocyclyl selected from the group consisting of furyl, thienyl, imidazolyl, pyrazolyl, isoxazolyl, thiazolyl, pyridyl, pyrazinyl and pyrimidinyl, unsubstituted or substituted by $C_1$-$C_4$-alkyl, di-$C_1$-$C_4$-alkyl or phenyl; 2-furylvinyl; 2-thienylvinyl; or phenyl substituted by one or two residues selected from $C_1$-$C_4$-alkyl, $C_1$-$C_4$-alkoxy, fluoro, chloro and trifluoromethyl; and B is phenyl unsubstituted or substituted by one or two residues selected from $C_1$-$C_4$-alkyl, $C_1$-$C_4$-alkoxy, fluoro, chloro and trifluoromethyl, or is pyridyl or thiazolyl; or an enol tautomer, lower alkyl enol ether or salt thereof.

5. A compound as claimed in claim 1 of formula I wherein A is 2- or 3-furyl, 5-bromo-2-furyl, 2- or 3-thienyl, 2-(2-furyl)vinyl, or 2-(2-thienyl)vinyl; and B is phenyl or phenyl substituted by one or two residues selected from $C_1$-$C_4$-alkyl, $C_1$-$C_4$-alkoxy, fluoro, chloro and trifluoromethyl, or is 2-pyridyl or 2-thiazolyl; or an enol tautomer or salt thereof.

6. A compound as claimed in claim 1 of formula I wherein A is 2- or 4-imidazolyl, 1-methyl-2- or 5-imidazolyl, 3-pyrazolyl, 1,5-dimethyl-3-pyrazolyl, 1,3-dimethyl-5-pyrazolyl, 3- or 5-isoxazolyl, 5-methyl-3-isoxazolyl, 3-methyl-5-isoxazolyl, 2-, 4- or 5-thiazolyl, 2-methyl- or phenyl-4-thiazolyl, 2,4-dimethyl-5-thiazolyl, or 2-, 3- or 4-pyridyl, and B is phenyl unsubstituted or substituted by one or two residues selected from $C_1$-$C_4$-alkyl, $C_1$-$C_4$-alkoxy, fluoro, chloro and trifluoromethyl, or an enol tautomer or salt thereof.

7. A compound as claimed in claim 1 of formula I wherein A is phenyl substituted by $C_1$-$C_4$-alkyl, $C_1$-$C_4$-alkoxy, fluoro or chloro, and B is phenyl unsubstituted or substituted by one or two residues selected from $C_1$-$C_4$-alkyl, $C_1$-$C_4$-alkoxy, fluoro, chloro and trifluoromethyl, or wherein A is phenyl and B is phenyl substituted by one or two residues selected from fluoro and chloro, or an enol tautomer or salt thereof.

8. A compound as claimed in claim 1 of formula I wherein A is 2-furyl or 2-thienyl and B is phenyl, chlorophenyl, fluorophenyl, trifluoromethylphenyl, dichlorophenyl, difluorophenyl, or chlorofluorophenyl, or an enol tautomer or salt thereof.

9. The compound as claimed in claim 1 of formula I wherein A is 2-thienyl and B is 3,4-dichlorophenyl, or an enol tautomer or salt thereof.

10. The compound as claimed in claim 1 of formula I wherein A is 2-thienyl and B is m-trifluoromethylphenyl, or an enol tautomer or salt thereof.

11. The compound as claimed in claim 1 of formula I wherein A is 2-thienyl and B is 3-chloro-4-fluorophenyl, or an enol tautomer or salt thereof.

12. The compound as claimed in claim 1 of formula I wherein A is 2-furyl and B is 3-chloro-4-fluorophenyl, or an enol tautomer or salt thereof.

13. The compound as claimed in claim 1 of formula I wherein A is 2-furyl and B is 2,4-difluorophenyl, or an enol tautomer or salt thereof.

14. A pharmaceutical preparation containing a compound of formula I of claim 1 or an enol tautomer, lower alkyl enol ether or salt thereof and customary carriers.

15. A compound of the formula I of claim 1 or an enol tautomer, lower alkyl enol ether or salt thereof for use in a method for the therapeutic treatment of the human or animal body.

16. The compounds of the formula I according to claim 1 or their enol tautomers, lower alkyl enol ethers and salts thereof for use as anti-inflammatory and antiarthritic active agents.

17. The use of compounds of formula I according to claim 1 or their enol tautomers, lower alkyl enol ethers and salts thereof and customary carriers for producing pharmaceutical preparations.

18. A process for the manufacture of a compound of formula I defined in claim 1, or an enol tautomer, lower alkyl ether or salts thereof, comprising

(a) condensing a compound of formula

$A\text{-}CO\text{-}CH_2CN$ (II),

or a salt thereof, with an isocyanate of formula

B-N=C=O (III),

wherein A and B have the meaning as previously defined; or

(b) condensing a compound of formula

CN
|
A-CO- CH -COOH (IV),

or a functional carboxyl derivative thereof, with an amine of formula

$B\text{-}NH_2$ (V),

wherein A and B have the meaning as previously defined; or

(c) condensing a carboxylic acid of formula

A-COOH (VI),

or a functional derivative thereof, with a compound of formula

$NC\text{-}CH_2\text{-}CONH\text{-}B$ (VII)

or a salt thereof, wherein A and B have the meaning as previously defined; or

(d) hydrolyzing an isoxazole of the formula

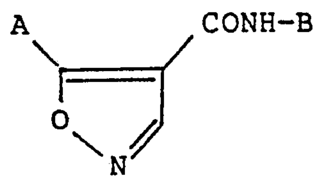

(VIII),

wherein A has the meaning as previously defined except isoxazolyl and B has the meaning as previously defined; or

(e) cleaving a conventional protecting group from a compound of the formula

CN
|
A′-CO- CH -CONH-B′ (IX),

wherein A′ has the meaning of A, represents five-membered nitrogen-containing heterocyclyl wherein the hydrogen atom bound to the nitrogen atom is replaced by a conventional amino protecting group, or represents hydroxy-substituted phenyl wherein the hydroxy group is protected by a conventional protecting group; and wherein B′ has the meaning of B or represents hydroxy-substituted phenyl wherein the hydroxy group is protected by a conventional protecting group; and wherein at least one of A′ and B′ are different from A and B, respectively; and optionally converting a resulting compound of formula I into another compound of formula I according to the definition; and optionally converting a resulting compound of formula I into a lower alkyl enol ether thereof, or converting a resulting compound of formula I into a salt thereof, or converting a resulting salt of a compound of formula I into the free compound or into another salt thereof.

Claims for the following Contracting States : ES , GR

CN
|
1. A process for the preparation of a compound of formula A-CO- CH -CONH-B (I),

wherein A is selected from the group consisting of five- and six-membered unsubstituted or substituted aromatic heterocyclyl with the exception of pyrrolyl, five- and six-membered aromatic heterocyclyl-vinyl, and unsubstituted and substituted phenyl, and wherein B is unsubstituted or substituted phenyl, pyridyl or thiazolyl, with the proviso that when A is unsubstituted phenyl B does not represent unsubstituted phenyl, methylphenyl or methoxyphenyl; or an enol tautomer, lower alkyl enol ether or salt thereof, comprising

(a) condensing a compound of formula

$A{-}CO{-}CH_2CN$ (II),

or a salt thereof, with an isooyanate of formula

$B{-}N{=}C{=}O$ (III),

wherein A and B have the meaning as previously defined; or

(b) condensing a compound of formula

$A{-}CO{-}\overset{CN}{\overset{|}{C}}H{-}COOH$ (IV),

or a functional carboxyl derivative thereof, with an amine of formula

$B{-}NH_2$ (V),

wherein A and B have the meaning as previously defined; or

(c) condensing a carboxylic acid of formula

A-COOH (VI),

or a functional derivative thereof, with a compound of formula

$NC{-}CH_2{-}CONH{-}B$ (VII)

or a salt thereof, wherein A and B have the meaning as previously defined; or

(d) hydrolyzing an isoxazole of the formula

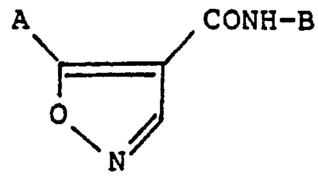

(VIII),

wherein A has the meaning as previously defined except isoxazolyl and B has the meaning as previously defined; or

(e) cleaving a conventional protecting group from a compound of the formula

$A'{-}CO{-}\overset{CN}{\overset{|}{C}}H{-}CONH{-}B'$ (IX),

wherein A′ has the meaning of A, represents five-membered nitrogen-containing heterocyclyl wherein the hydrogen atom bound to the nitrogen atom is replaced by a conventional amino protecting group, or represents hydroxy-substituted phenyl wherein the hydroxy group is protected by a conventional protecting group; and wherein B′ has the meaning of B or represents hydroxy-substituted phenyl wherein the hydroxy group is protected by a conventional protecting group; and wherein at least one of A′ and B′ are different from A and B, respectively; and optionally converting a resulting compound of formula I into another compound of formula I according to the definition; and optionally converting a resulting compound of formula I into a lower alkyl enol ether thereof, or

converting a resulting compound of formula I into a salt thereof, or converting a resulting salt of a compound of formula I into the free compound or into another salt thereof.

2. A process according to claim 1 wherein there are produced compounds of the formula I given in claim 1 wherein

A is selected from the group consisting of five membered aromatic heterocyclyl comprising one oxygen atom, one sulfur atom, or two heteroatoms wherein one is nitrogen and the other one is nitrogen, oxygen or sulfur, and wherein the aromatic heterocycle is unsubstituted or substituted at carbon atoms by one, two or three residues selected from lower alkyl, phenyl, furyl, thienyl and halogen, and at a nitrogen atom by lower alkyl; six-membered aromatic heterocyclyl comprising one or two nitrogen atoms, unsubstituted or substituted at carbon atoms by lower alkyl or phenyl; furylvinyl; thienylvinyl; and phenyl substituted by one or two residues selected from lower alkyl, phenyl, hydroxy, lower alkoxy, halogen and trifluoromethyl; and

B is selected from the group consisting of phenyl; phenyl substituted by one or two residues selected from lower alkyl, phenyl, hydroxy, lower alkoxy, halogen and trifluoromethyl; pyridyl; and thiazolyl;

or an enol tautomer, lower alkyl enol ether or salt thereof.

3. A process according to claim 1 wherein there are produced compounds of the formula I given in claim 1 wherein A is phenyl and B is phenyl substituted by one or two residues selected from halogen and trifluoromethyl, or an enol tautomer, lower alkyl enol ether or salt thereof.

4. A process according to claim 1 wherein there are produced compounds of the formula I given in claim 1 wherein A is five- or six-membered aromatic heterocyclyl selected from the group consisting of furyl, thienyl, imidazolyl, pyrazolyl, isoxazolyl, thiazolyl, pyridyl, pyrazinyl and pyrimidinyl, unsubstituted or substituted by $C_1$-$C_4$-alkyl, di-$C_1$-$C_4$-alkyl or phenyl; 2-furylvinyl; 2-thienylvinyl; or phenyl substituted by one

or two residues selected from $C_1$-$C_4$-alkyl, $C_1$-$C_4$-alkoxy, fluoro, chloro and trifluoromethyl; and B is phenyl unsubstituted or substituted by one or two residues selected from $C_1$-$C_4$-alkyl, $C_1$-$C_4$-alkoxy, fluoro, chloro and trifluoromethyl, or is pyridyl or thiazolyl; or an enol tautomer, lower alkyl enol ether or salt thereof.

5. A process according to claim 1 wherein there are produced compounds of the formula I given in claim 1 wherein A is 2- or 3-furyl, 5-bromo-2-furyl, 2- or 3-thienyl, 2-(2-furyl)vinyl, or 2-(2-thienyl)vinyl; and B is phenyl or phenyl substituted by one or two residues selected from $C_1$-$C_4$-alkyl, $C_1$-$C_4$-alkoxy, fluoro, chloro and trifluoromethyl, or is 2-pyridyl or 2-thiazolyl; or an enol tautomer or salt thereof.

6. A process according to claim 1 wherein there are produced compounds of the formula I given in claim 1 wherein A is 2- or 4-imidazolyl, 1-methyl-2- or 5-imidazolyl, 3-pyrazolyl, 1,5-dimethyl-3-pyrazolyl, 1,3-dimethyl-5-pyrazolyl, 3- or 5-isoxazolyl, 5-methyl-3-isoxazolyl, 3-methyl-5-isoxazolyl, 2-, 4- or 5-thiazolyl, 2-methyl- or phenyl-4-thiazolyl, 2,4-dimethyl-5-thiazolyl, or 2-, 3- or 4-pyridyl, and B is phenyl unsubstituted or substituted by one or two residues selected from $C_1$-$C_4$-alkyl, $C_1$-$C_4$-alkoxy, fluoro, chloro and trifluoromethyl, or an enol tautomer or salt thereof.

7. A process according to claim 1 wherein there are produced compounds of the formula I given in claim 1 wherein A is phenyl substituted by $C_1$-$C_4$-alkyl, $C_1$-$C_4$-alkoxy, fluoro or chloro, and B is phenyl unsubstituted or substituted by one or two residues selected from $C_1$-$C_4$-alkyl, $C_1$-$C_4$-alkoxy, fluoro, chloro and trifluoromethyl, or wherein A is phenyl and B is phenyl substituted by one or two residues selected from fluoro and chloro, or an enol tautomer or salt thereof.

8. A process according to claim 1 wherein there are produced compounds of the formula I given in claim 1 wherein A is 2-furyl or 2-thienyl and B is phenyl, chlorophenyl, fluorophenyl, trifluoromethylphenyl, dichlorophenyl, difluorophenyl, or chlorofluorophenyl, or an enol tautomer or salt thereof.

9. A process according to claim 1 wherein there is produced a compound of formula I given in claim 1 wherein A is 2-thienyl and B is 3,4-dichlorophenyl, or an enol tautomer or salt thereof.

10. A process according to claim 1 wherein there is produced a compound of formula I given in claim 1 wherein A is 2-thienyl and B is m-trifluoromethylphenyl, or an enol tautomer or salt thereof.

11. A process according to claim 1, wherein there is produced a compound of formula I given in claim 1 wherein A is 2-thienyl and B is 3-chloro-4-fluorophenyl, or an enol tautomer or salt thereof.

12. A process according to claim 1, wherein there is produced a compound of formula I given in claim 1 wherein A is 2-furyl and B is 3-chloro-4-fluorophenyl, or an enol tautomer or salt thereof.

13. A process according to claim 1, wherein there is produced a compound of formula I given in claim 1 wherein A is furyl and B is 2,4-difluorophenyl, or an enol tautomer or salt thereof.